# EUROPEAN PATENT SPECIFICATION

(11) **EP 2 859 857 B1**
(45) Date of publication and mention of the grant of the patent: **25.10.2017**
(21) Application number: 13800451.0
(22) Date of filing: 06.06.2013
(51) Int. Cl.: A61B 17/32

(54) **CONNECTION MECHANISM FOR ULTRASOUND-VIBRATION GENERATING/TRANSMITTING UNIT, AND PRODUCTION METHOD FOR ULTRASOUND-VIBRATION GENERATING/TRANSMITTING UNIT**
VERBINDUNGSMECHANISMUS FÜR EINE ULTRASOUND-SCHWINGUNGSERZEUGUNGS/-ÜBERTRAGUNGSEINHEIT UND HERSTELLUNGSVERFAHREN FÜR EINE ULTRASCHALL-SCHWINGUNGSERZEUGUNGS/-ÜBERTRAGUNGSEINHEIT
MÉCANISME DE CONNEXION D'UNITÉ DE GÉNÉRATION/TRANSMISSION DE VIBRATIONS ULTRASONORES, ET PROCÉDÉ DE FABRICATION D'UNITÉ DE GÉNÉRATION/TRANSMISSION DE VIBRATIONS ULTRASONORES

(30) Priority: 06.06.2012 US 201261656160 P
(43) Date of publication of application: 15.04.2015
(73) Proprietor: Olympus Corporation, Hachioji-shi, Tokyo 192-8507 (JP)
(72) Inventor: AKAGANE, Tsunetaka, Hachioji-shi, Tokyo 192-8512 (JP)
(74) Representative: Gunzelmann, Rainer
(86) International application number: PCT/JP2013/065715
(87) International publication number: WO 2013/183713

(56) References cited:
- WO-A2-2011/110774
- JP-A- H08 224 252
- JP-A- 2010 034 817
- JP-A- 2012 039 304
- JP-U- S5 987 810
- US-A1- 2001 017 448
- US-B1- 6 204 592

## Description

### Technical Field

The present invention relates to a connection mechanism for an ultrasonic-vibration generating/transmitting unit which generates and transmits ultrasonic vibration, and a production method for the ultrasonic-vibration generating/transmitting unit.

### Background Art

In general, an ultrasonic treatment apparatus includes an ultrasonic vibrator which generates ultrasonic vibration, a probe which is connected to the ultrasonic vibrator and which is a transmitting member to transmit the ultrasonic vibration generated in the ultrasonic vibrator, and a treatment portion which is provided at the end of the probe and which treats, for example, a treatment part of a living tissue with the ultrasonic vibration transmitted by the probe. The ultrasonic vibrator and the probe serve as an ultrasonic-vibration generating/transmitting unit which generates ultrasonic vibration and which transmits the ultrasonic vibration. The ultrasonic vibrator represents one of parts constituting the ultrasonic-vibration generating/transmitting unit, and the probe represents the other part.

The ultrasonic vibrator and the probe need to be firmly coupled to each other so that the ultrasonic vibration generated in the ultrasonic vibrator is fully transmitted to the probe. The probe including the treatment portion that has been once used needs to be replaced with a probe including a clean treatment portion for each surgical operation. Therefore, the probe is removable from the ultrasonic vibrator unit.

For example, Patent Literature 1 has disclosed a surgical handpiece which is an example of the above ultrasonic treatment apparatus.

In the surgical handpiece according to Patent Literature 1, a joint portion and a chip portion are removable from each other. When the joint portion and the chip portion are connected to each other, a depression portion provided at the front end of the joint portion is first fitted to a projection provided at the rear end of the chip portion. A fixing thread is then fastened to a connection thread provided on the outer circumference of the distal end portion of the joint portion. As a result, a joint surface between the joint portion and the chip portion, and a joint surface between the chip portion and the fixing thread are firmly press-connected, and the joint portion and the chip portion are connected to each other.

When the joint portion and the chip portion are disconnected from each other, the fixing thread is separated from the connection thread provided on the outer circumference of the distal end portion of the joint portion.

Patent Literature 2 discloses an ultrasonic vibrator comprising a piezoelectric element unit, a front member, including respective support portions, and a casing, including protrusion portions defined by grooves. The support portions are inserted into the grooves and along the same grooves.

Patent Literature 3 discloses a vibratory instrument comprising a tool carrier and a tool, where the tool carrier is a single-piece part that has a body with a first end or base for fastening rigidly to a handpiece, that is a generator of sound or ultrasound vibration, and may include a transducer, for example made of a piezoelectric material, and mechanically coupled in a rigid manner to the insert, so as to transmit vibratory waves thereto. The tool carrier is rigidly fastened to the handpiece by being screwed onto a fastener element secured to the transducer of the handpiece; for this purpose, the base of the tool carrier includes a recess having tapping formed in the wall thereof and suitable for co-operating with a thread present on the element. The tool carrier may also be permanently fastened to the handpiece, e.g. by welding, or it may correspond merely to an extension thereof. The tool carrier has a curved shape to facilitate access to the site for treatment.

Patent Literature 4 discloses a cutting tool attaching and detaching apparatus of ultrasonic vibration machine device comprising a collet, a collet attaching device, a collet removing device, and a collet removing piece, that is provided with a C-shaped engaging piece which can be engaged with and removed from a ring groove of the collet from a lateral direction. The collet removing device has a screw portion meshed with a screw portion of a shank, and an abutting surface which can abut with an end surface of the collet removing piece engaged with and attached to the collet. The collet removing device can remove the collet from the taper hole of the shank via the collet removing piece due to a screwing operation against the shank; the collet removing piece can be immediately separated from the collet and the collet removing device can be removed from the shank, after removing the collet from the taper hole of the shank.

### Citation List

### Patent Literature

Patent Literature 1: Jpn. Pat. Appln. KOKAI Publication No. 8-224252
Patent Literature 2: Jpn. Pat. Appln. Publication No. 2012 039304 A
Patent Literature 3: International Pat. Appln. Publication No. 2011/110774 A2
Patent Literature 4: US Pat. Appln. Publication No. 2001/017448 A1

### Summary of Invention

### Technical Problem

According to Patent Literature 1, when the joint portion and the chip portion are connected to each other, the joint surface between the joint portion and the chip portion, and the joint surface between the chip portion and the fixing thread need to be firmly press-connected. Therefore, the fixing thread needs to be more toothed to the connection thread of the joint portion. In other words, the fixing thread needs to be rotated relative to the connection thread of the joint portion more than once. On the other hand, when the joint portion and the chip portion are disconnected from each other, the fixing thread needs to be separated from the connection thread. Therefore, if the connection thread of the joint portion and the fixing thread are toothed in more parts, it takes time for the fixing thread to be separated. In this way, according to Patent Literature 1, the connection operation and the disconnection operation are troublesome. In view of the above-mentioned problems, the present invention provides a connection mechanism for an ultrasonic-vibration generating/transmitting unit and a production method for the ultrasonic-vibration generating/transmitting unit which enable one of parts constituting the ultrasonic-vibration generating/transmitting unit and the other part to be connected to and disconnected from each other without any trouble.

### Solution to Problem

The problem is solved by a connection mechanism for an ultrasonic-vibration generating/transmitting unit according to appended independent claim 1 and a production method for the ultrasonic-vibration generating/transmitting unit according to appended independent claim 11. Preferred embodiments are given in the dependent claims. An aspect of a connection mechanism for an ultrasonic-vibration generating/transmitting unit, the connection mechanism being provided in each of parts constituting the ultrasonic-vibration generating/transmitting unit which generates and transmits ultrasonic vibration, the connection mechanism connecting the parts to each other to form the ultrasonic-vibration generating/transmitting unit, the connection mechanism including one end portion representing one end portion of one of the parts; a held portion which is provided on the outer circumferential surface of the one end portion and which is provided along the circumferential direction of the one end portion; other end portion representing one end portion of the other part which is connected to the one end portion; other connection portion which is provided on the outer circumferential surface of the other end portion and which is provided over the circumference of the other end portion along the circumferential direction of the other end portion; and a cylinder member, the cylinder member including a cylinder-side one end portion into which the one end portion is inserted, a cylinder-side other end portion into which the other end portion is inserted, a cylinder-side protrusion portion which is provided on the inner circumferential surface of the cylinder-side one end portion and which protrudes inward from the inner circumferential surface and which is provided along the circumferential direction of the cylinder-side one end portion, and a cylinder-side connection portion which is provided on the inner circumferential surface of the cylinder-side other end portion and which is provided over the circumference of the cylinder-side other end portion along the circumferential direction of the cylinder-side other end portion, wherein when the one end portion is inserted into the cylinder member from the cylinder-side one end portion and when the other end portion is inserted into the cylinder member from the cylinder-side other end portion so that the other connection portion is connected to the cylinder-side connection portion, the held portion is held by the end face of the other end portion and the cylinder-side protrusion portion in the axial direction of the cylinder member, whereby the one end portion and the other end portion are connected to each other.

An aspect of a production method for an ultrasonic-vibration generating/transmitting unit to connect parts constituting the ultrasonic-vibration generating/transmitting unit which generates and transmits ultrasonic vibration to form the ultrasonic-vibration generating/transmitting unit, wherein there are provided: one end portion representing one end portion of one of the parts; a held portion which is provided on the outer circumferential surface of the one end portion and which is provided along the circumferential direction of the one end portion; other end portion representing one end portion of the other part which is connected to the one end portion; other connection portion which is provided on the outer circumferential surface of the other end portion and which is provided over the circumference of the other end portion along the circumferential direction of the other end portion; and a cylinder member, the cylinder member including a cylinder-side one end portion into which the one end portion is inserted, a cylinder-side other end portion into which the other end portion is inserted, a cylinder-side protrusion portion which is provided on the inner circumferential surface of the cylinder-side one end portion and which protrudes inward from the inner circumferential surface and which is provided along the circumferential direction of the cylinder-side one end portion, and a cylinder-side connection portion which is provided on the inner circumferential surface of the cylinder-side other end portion and which is provided over the circumference of the cylinder-side other end portion along the circumferential direction of the cylinder-side other end portion, the production method including a process of connecting one of the part and the other part, the connection process including inserting one end portion into the cylinder member from the cylinder-side one end portion so that the held portion is held by the end face of the other end portion and the cylinder-side protrusion portion in the axial direction of the cylinder member, inserting the other end portion into the cylinder member from the cylinder-side other end portion so that the other connection portion is connected to the cylinder-side connection portion, and thereby connecting the one end portion and the other end portion to each other.

### Advantageous Effects of Invention

According to the present invention, it is possible to provide a connection mechanism for an ultrasonic-vibration generating/transmitting unit and a production method for the ultrasonic-vibration generating/transmitting unit which enable one of parts constituting the ultrasonic-vibration generating/transmitting unit and the other part to be connected to and disconnected from each other without any trouble.

### Brief Description of Drawings

FIG. 1 is a schematic diagram of an ultrasonic treatment system according to a first embodiment of the present invention;
FIG. 2 is a schematic diagram showing the configuration of an ultrasonic treatment apparatus;
FIG. 3 is an exploded perspective view of an ultrasonic-vibration generating/transmitting unit and a connection mechanism;
FIG. 4A is a perspective view of one end portion of an ultrasonic vibrator;
FIG. 4B is a side view of one end portion of the ultrasonic vibrator;
FIG. 4C is a front view of one end portion of the ultrasonic vibrator;
FIG. 5A is a front view of a cylinder member viewed from a cylinder-side one end portion;
FIG. 5B is a sectional view taken along the line 5B-5B shown in FIG. 5A;
FIG. 6 is a flowchart showing a production method for the ultrasonic-vibration generating/transmitting unit according to the first embodiment;
FIG. 7A is a diagram illustrating Step 1 shown in FIG. 6;
FIG. 7B is a diagram illustrating Step 1 shown in FIG. 6;
FIG. 7C is a diagram illustrating Step 2 shown in FIG. 6;
FIG. 7D is a diagram illustrating Step 2 shown in FIG. 6;
FIG. 7E is a diagram illustrating Step 2 shown in FIG. 6;
FIG. 7F is a diagram illustrating Step 3 shown in FIG. 6;
FIG. 7G is a diagram illustrating Step 3 shown in FIG. 6;
FIG. 7H is a diagram illustrating Step 3 shown in FIG. 6;
FIG. 7I is a diagram illustrating Step 3 shown in FIG. 6;
FIG. 7J is a diagram illustrating Step 4 shown in FIG. 6;
FIG. 7K is a diagram illustrating Step 4 shown in FIG. 6;
FIG. 7L is a diagram illustrating Step 5 shown in FIG. 6;
FIG. 7M is a diagram illustrating Step 5 shown in FIG. 6;
FIG. 8 is a diagram illustrating a modification of one end portion of the ultrasonic vibrator;
FIG. 9 is an exploded perspective view of an ultrasonic-vibration generating/transmitting unit and a connection mechanism according to a second embodiment;
FIG. 10 is a flowchart showing a production method for the ultrasonic-vibration generating/transmitting unit according to the first embodiment;
FIG. 11A is a diagram illustrating Step 11 shown in FIG. 10;
FIG. 11B is a diagram illustrating Step 11 shown in FIG. 10;
FIG. 11C is a diagram illustrating Step 12 shown in FIG. 10;
FIG. 11D is a diagram illustrating Step 12 shown in FIG. 10; and
FIG. 11E is a diagram illustrating Step 13 shown in FIG. 10.

Brief Description of Embodiments Hereinafter, embodiments of the present invention will be described in detail with reference to the drawings.

### [First Embodiment]

### [Configuration]

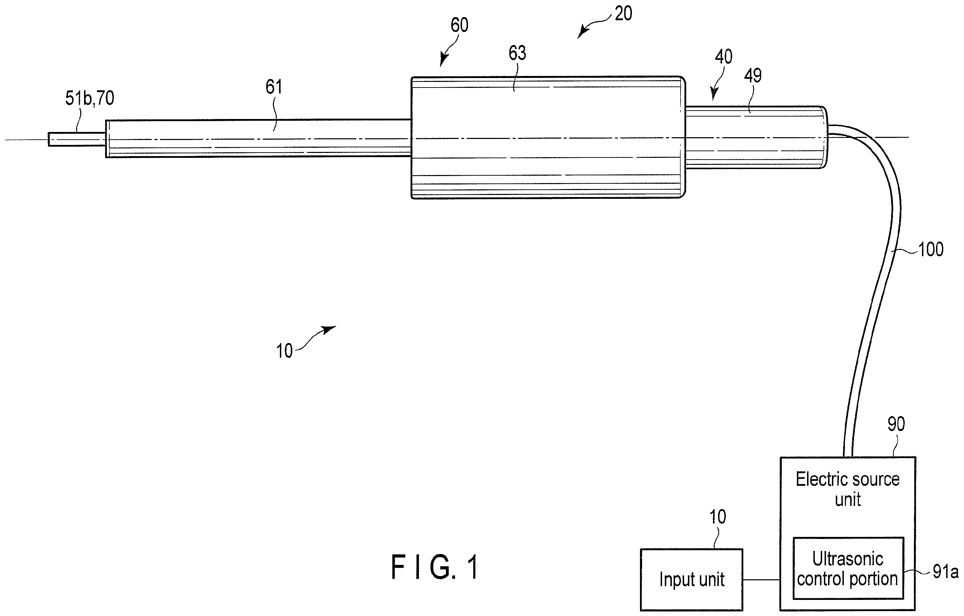

The first embodiment is described with reference to FIG. 1, FIG. 2, FIG. 3, FIG. 4A, FIG. 4B, FIG. 4C, FIG. 5A, FIG. 5B, FIG. 6, FIG. 7A, FIG. 7B, FIG. 7C, FIG. 7D, FIG. 7E, FIG. 7F, FIG. 7G, FIG. 7H, FIG. 7I, FIG. 7J, FIG. 7K, FIG. 7L, and FIG. 7M. In some of the drawings, some components are not shown for clarity.

### [Ultrasonic Treatment System (hereinafter, treatment system 10)]

As shown in FIG. 1, the treatment system 10 includes an ultrasonic treatment apparatus (hereinafter, treatment apparatus 20) which treats a treatment part such as a living tissue with ultrasonic vibration, and an electric source unit 90 which generates an electric current for the ultrasonic vibration of the treatment apparatus 20. The treatment system 10 further includes a cable 100 which supplies an electric current generated in the electric source unit 90 to the treatment apparatus 20 from the electric source unit 90 when one end of a cable 100a is connected to the treatment apparatus 20 and the other end portion of the cable 100a is connected to the electric source unit 90, and an input unit 110 such as a foot switch.

### [Treatment Apparatus 20]

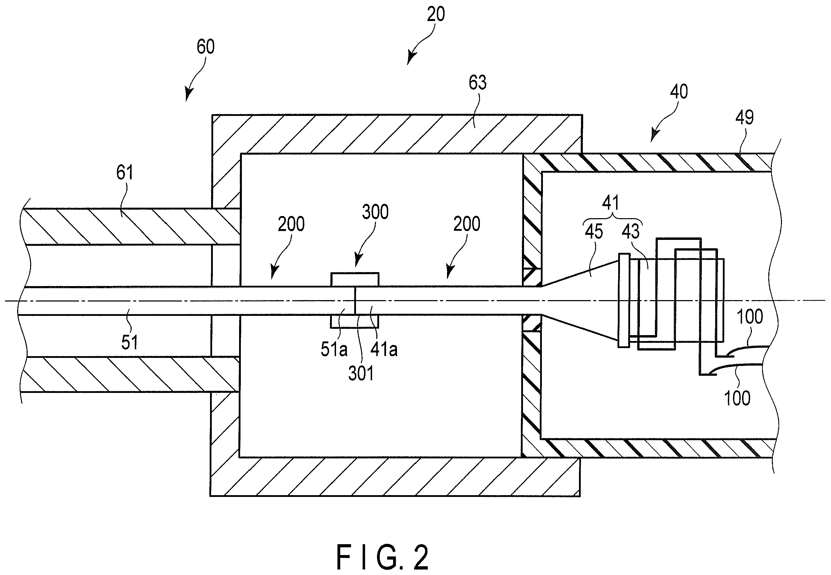

As shown in FIG. 1 and FIG. 2, the treatment apparatus 20 includes an ultrasonic vibrator unit (hereinafter, vibrator unit 40) which generates ultrasonic vibration by the electric current supplied from the electric source unit 90 via the cable 100, and an ultrasonic probe (hereinafter, probe 51) which is connected to the vibrator unit 40 and which is a transmission member to transmit the ultrasonic vibration generated in the vibrator unit 40. The treatment apparatus 20 further includes a sheath unit 60 into which the probe 51 is inserted, and a treatment portion 70 which treats the treatment part with the ultrasonic vibration transmitted by the probe 51.

### [Vibrator Unit 40]

As shown in FIG. 1 and FIG. 2, the vibrator unit 40 includes an ultrasonic vibrator (hereinafter, vibrator 41) which generates ultrasonic vibration, and a case 49 which houses the vibrator 41.

As shown in FIG. 2, the vibrator 41 includes a piezoelectric element 43 which converts the electric current supplied from the electric source unit 90 to ultrasonic vibration, and a horn 45 which amplifies the amplitude of the ultrasonic vibration generated in the piezoelectric element 43.

As shown in FIG. 2, the piezoelectric element 43 has, for example, a ring-shape, and more than one piezoelectric element 43 is provided. The piezoelectric elements 43 are in close contact with one another along the longitudinal axis direction of the vibrator 41 (probe 51). The piezoelectric element 43 is connected to the cable 100.

As shown in FIG. 2, the horn 45 has, for example, a columnar shape, and is connected to the piezoelectric element 43. The horn 45 is attached to the case 49 so that the distal end portion of the horn 45 protrudes from the case 49. The horn 45 is an amplitude increasing portion which increases the amplitude of ultrasonic vibration.

This vibrator 41 is, for example, a bolt-clamped Langevin type transducer (BLT).

### [Probe 51]

As shown in FIG. 2, the probe 51 is formed by an elongated member having a longitudinal axis. This probe 51 has the same thickness as, for example, that of the distal end portion of the horn 45. As shown in FIG. 2, the probe 51 includes one end portion 51a which is connected to one end portion 41a of the vibrator 41 representing the distal end portion of the horn 45, and the other end portion 51b which transmits ultrasonic vibration to the treatment part as shown in FIG. 1. The ultrasonic vibration is transmitted to the one end portion 51a from the vibrator 41. The ultrasonic vibration is then transmitted to the side of the other end portion 51b of the probe 51 from the one end portion 51a of the probe 51. The treatment portion 70 is formed in the other end portion 51b of the probe 51.

### [Sheath Unit 60]

As shown in FIG. 1 and FIG. 2, the sheath unit 60 includes a sheath 61 through which the probe 51 is inserted, and a holding case 63 which is provided between the case 49 and the sheath 61 and which is coupled to the case 49 and which holds the case 49 and the sheath 61.

The sheath 61 has, for example, a cylindrical shape. The sheath 61 includes one end portion connected to the holding portion 63 as shown in FIG. 2, and the other end portion from which the other end portion 51b of the probe 51 protrudes as shown in FIG. 1.

The holding portion 63 has, for example, a cylindrical shape. The holding portion 63 includes a one end portion connected to the one end portion of the sheath 61, and the other end portion connected to the case 49. For example, inside the holding portion 63, the one end portion 51a of the probe 51 and the one end portion 41a of the vibrator 41 are connected to each other. In this case, the one end portion 51a of the probe 51 is inserted from the one end portion side of the holding portion 63, and the one end portion 41a of the vibrator 41 is inserted from the other end portion side of the holding portion 63.

### [Treatment Portion 70]

As shown in FIG. 1, the treatment portion 70 includes the other end portion 51b of the probe 51 which ultrasonically vibrates.

### [Electric Source Unit 90]

The electric source unit 90 includes an ultrasonic control portion 91a which is connected to the input unit 110 and which controls an electric current for ultrasonic vibration in accordance with an input amount of the input unit 110.

### [Ultrasonic-vibration Generating/Transmitting Unit (hereinafter, Generating/Transmitting Unit 200) - Connection Mechanism 300]

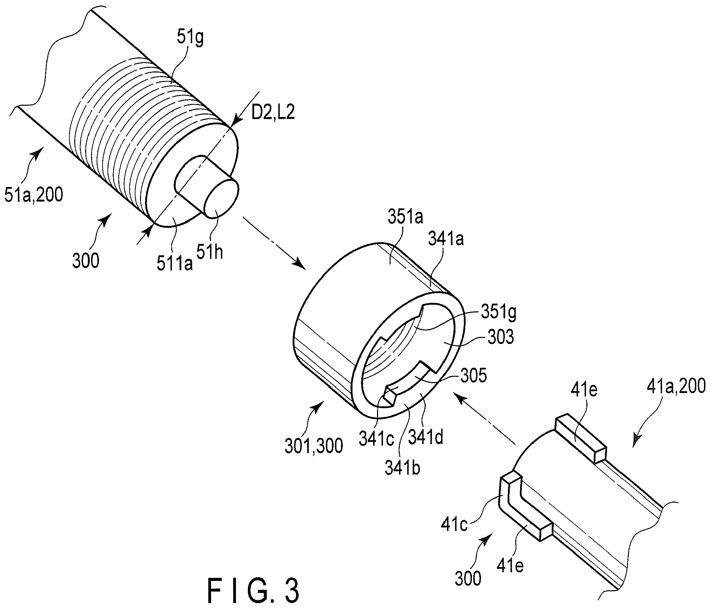

As shown in FIG. 2 and FIG. 3, the treatment apparatus 20 further includes a generating/transmitting unit 200 which is constituted by parts connected to one another, generates ultrasonic vibration and transmits ultrasonic vibration to the treatment portion 70, and a connection mechanism 300 which is provided in each of parts constituting the generating/transmitting unit 200 and which connects the parts to one another to constitute the generating/transmitting unit 200.

### [Generating/Transmitting Unit 200]

As shown in FIG. 2 and FIG. 3, the generating/transmitting unit 200 includes, for example, the vibrator 41 and the probe 51 that have been described above, according to the present embodiment. In this case, the vibrator 41 represents one of parts constituting the generating/transmitting unit 200, and the probe 51 represents the other part. In this way, the generating/transmitting unit 200 is constituted by more than one part.

### [Connection Mechanism 300 provided in Vibrator 41]

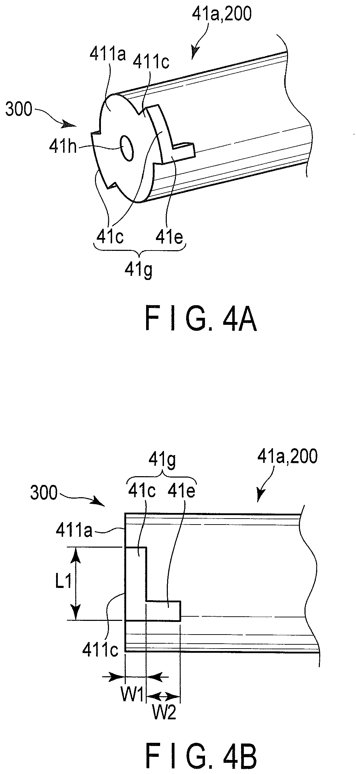

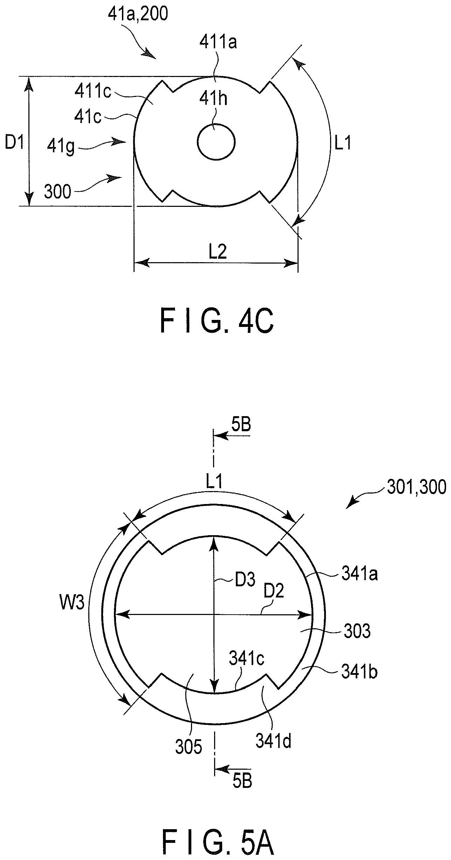

As shown in FIG. 3, FIG. 4A, FIG. 4B, and FIG. 4C, the connection mechanism 300 includes the columnar one end portion 41a of the vibrator 41, and a held portion 41c which is provided on the outer circumferential surface of the one end portion 41a of the vibrator 41 and which is provided along the circumferential direction of the one end portion 41a. The connection mechanism 300 further includes an abutment portion 41e which is provided on the outer circumferential surface of the one end portion 41a of the vibrator 41 and which is provided along, for example, the axial direction of the one end portion 41a and which comes into abutment with a later-described cylinder-side protrusion portion 341c. The one end portion 41a of the vibrator 41 is one end portion representing one end portion of one of parts.

As shown in FIG. 3 and FIG. 4A, the held portion 41c and the abutment portion 41e are protrusion portions diametrically protruding from the outer circumferential surface of the one end portion 41a. The held portion 41c and the abutment portion 41e are, for example, columnar. The held portion 41c and the abutment portion 41e are, for example, integrated with the one end portion 41a. One end portion of the held portion 41c and one end portion of the abutment portion 41e are, for example, connected to each other, and the held portion 41c and the abutment portion 41e are integrated with each other. The held portion 41c and the abutment portion 41e function as a positioning portion 41g which positions the one end portion 41a relative to a later-described cylinder member 301.

As shown in FIG. 4B, the positioning portion 41g is substantially L-shaped. As shown in FIG. 4A and FIG. 4C, two positioning portions 41g, for example, are provided, and are provided 180 degrees apart from each other in the circumferential direction. For example, the positioning portions 41g are provided rotationally symmetrically with respect to the central axis of the one end portion 41a.

As shown in FIG. 4A, FIG. 4B, and FIG. 4C, the one end portion 41a has an end face 411a provided at the extreme end in the axial direction of the vibrator 41. The held portion 41c has an end face 411c provided at the extreme end in the axial direction of the vibrator 41. The end face 411a and the end face 411c are formed in a plane that intersects at right angles with the axial direction of the one end portion 41a, and are flush. The end face 411a and the end face 411c are connected to each other, and are planes.

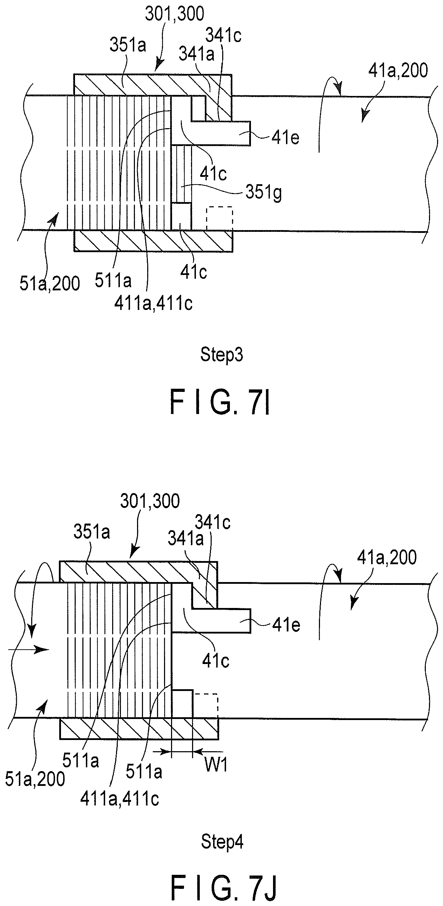

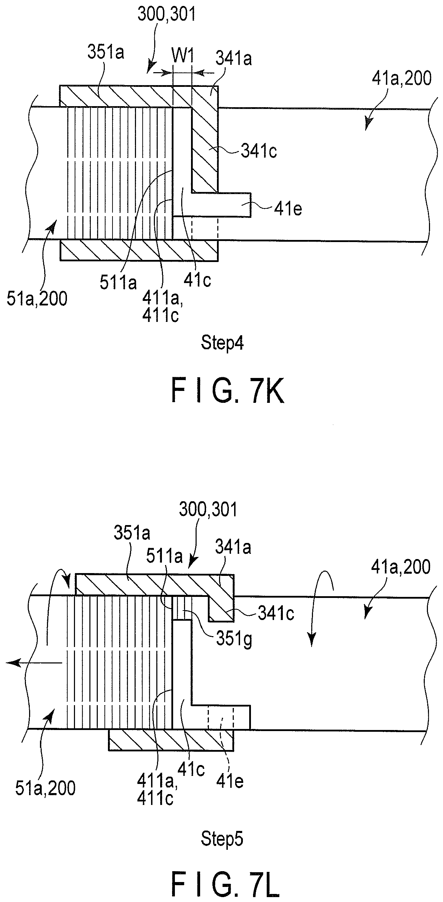

As shown in FIG. 7J and FIG. 7K, the held portion 41c is held between an end face 511a of the one end portion 51a of the probe 51 and the cylinder-side protrusion portion 341c in the axial direction of the cylinder member 301.

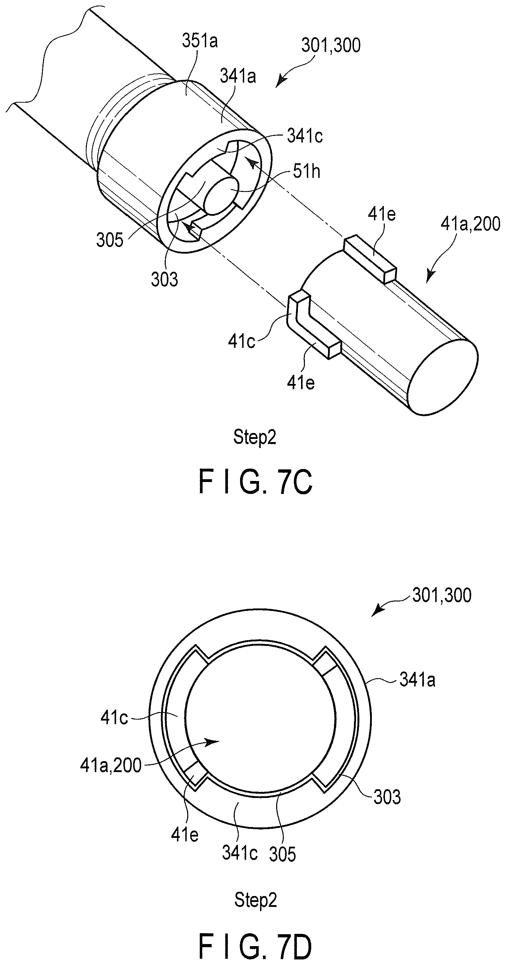

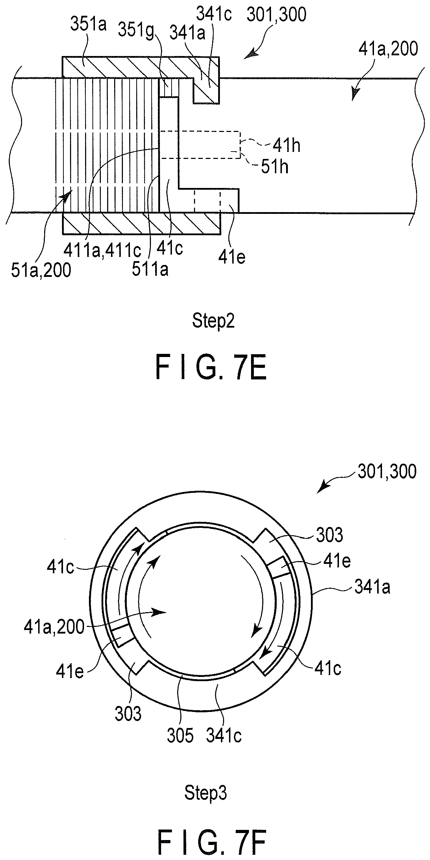

When the one end portion 41a of the vibrator 41 is inserted into the cylinder member 301 from a later-described cylinder-side one end portion 341a as shown in FIG. 7C, the abutment portion 41e is provided collinearly with the later-described cylinder-side protrusion portion 341c in the circumferential direction of the cylinder member 301 as shown in FIG. 7D and FIG. 7E. When the held portion 41c is held between the end face 511a of the one end portion 51a of the probe 51 and the cylinder-side protrusion portion 341c in the axial direction of the cylinder member 301 as shown in FIG. 7J and FIG. 7K, the abutment portion 41e is in abutment with the cylinder-side protrusion portion 341c in the axial direction of the cylinder member 301.

As shown in FIG. 4A and FIG. 4C, the connection mechanism 300 includes a depression portion 41h which is depressed in the end face 411a and which is provided on the central axis of the vibrator 41. The depression portion 41h is, for example, columnar.

As shown in FIG. 4B and FIG. 4C, the length of the held portion 41c in the circumferential direction of the one end portion 41a is L1. As shown in FIG. 4C, the length from one held portion 41c to the other held portion 41c in the diametrical direction of the one end portion 41a is L2. As shown in FIG. 4B, the width of the held portion 41c is W1 in the axial direction of the one end portion 41a. As shown in FIG. 4B, the width of the abutment portion 41e is W2 in the axial direction of the one end portion 41a. As shown in FIG. 4B and FIG. 4C, the diameter of the end face 411a is D1.

### [Connection Mechanism 300 provided in Probe 51]

As shown in FIG. 3, the connection mechanism 300 includes the columnar one end portion 51a of the probe 51 to be connected to the one end portion 41a of the vibrator 41, and a probe-side connection portion 51g which is provided on the outer circumferential surface of the one end portion 51a of the probe 51 and which is provided over, for example, the circumference of the one end portion 51a around the axis of the one end portion 51a. The one end portion 51a of the probe 51 is other end portion representing one end portion of the other part.

As shown in FIG. 2, the one end portion 51a has substantially the same thickness as the one end portion 41a of the vibrator 41. As shown in FIG. 2, when the vibrator 41 and the probe 51 are connected to each other, the central axis of the probe 51 including the one end portion 51a is provided coaxially with the central axis of the vibrator 41 including the one end portion 41a.

As shown in FIG. 3, the one end portion 51a has the end face 511a provided at the extreme end in the axial direction of the probe 51. The end face 511a is a plane formed in a plane that intersects at right angles with the axial direction of the one end portion 51a. The end face 511a is substantially the same size as the end face 411a. A diameter D2 of the end face 511a is, for example, substantially the same as the length L2.

The probe-side connection portion 51g is, for example, a thread groove, and is the other connection portion.

As shown in FIG. 3, the connection mechanism 300 further includes a protrusion portion 51h which is provided to protrude from the end face 511a and which is provided on the central axis of the probe 51 and which is fitted into the depression portion 41h. The protrusion portion 51h is, for example, columnar.

### [Components unique to Connection Mechanism 300]

As shown in FIG. 2 and FIG. 3, the connection mechanism 300 further includes the cylinder member 301.

As shown in FIG. 3, the cylinder member 301 has the cylinder-side one end portion 341a representing one end portion of the cylinder member 301 into which the one end portion 41a of the vibrator 41 is inserted, and a cylinder-side other end portion 351a representing the other end portion of the cylinder member 301 into which the one end portion 51a of the probe 51 is inserted.

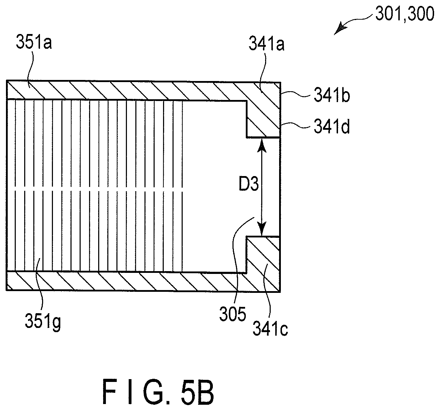

As shown in FIG. 3, FIG. 5A, and FIG. 5B, the cylinder member 301 has the cylinder-side protrusion portion 341c which is provided on the inner circumferential surface of the cylinder-side one end portion 341a and which protrudes to inside of the cylinder member 301 from the inner circumferential surface and which is provided along the circumferential direction of the cylinder-side one end portion 341a. The cylinder member 301 further includes a cylinder-side connection portion 351g which is provided on the inner circumferential surface of the cylinder-side other end portion 351a and which is provided over the circumference of the cylinder-side other end portion 351a along the circumferential direction of the cylinder-side other end portion 351a and which is connected to the probe-side connection portion 51g when the one end portion 51a of the probe 51 is inserted into the cylinder-side other end portion 351a.

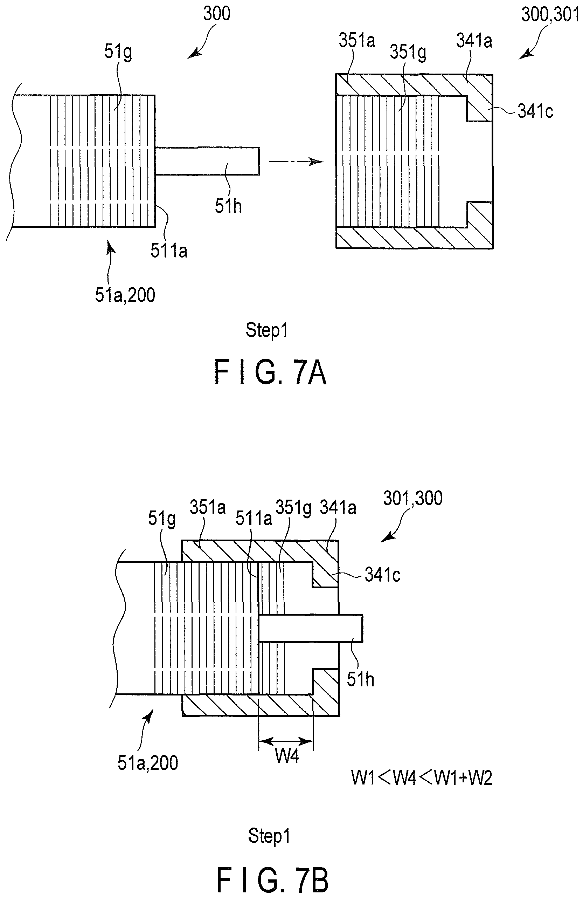

When the one end portion 51a of the probe 51 is inserted into the cylinder-side other end portion 351a as shown in FIG. 7A so that the cylinder-side connection portion 351g is connected to the probe-side connection portion 51g as shown in FIG. 7B, the cylinder-side protrusion portion 341c protrudes from the inner circumferential surface collinearly with part of the end face 511a in the axial direction of the cylinder member 301 as shown in FIG. 7B. As shown in FIG. 5A and FIG. 5B, the cylinder-side protrusion portion 341c is integrated with the cylinder-side one end portion 341a.

The cylinder-side protrusion portion 341c has only to be provided in the same manner as the held portion 41c. Thus, as shown in FIG. 3 and FIG. 5A, two cylinder-side protrusion portions 341c, for example, are provided, and are provided 180 degrees apart from each other in the circumferential direction. As shown in FIG. 5A, the length of the cylinder-side protrusion portion 341c in the circumferential direction of the cylinder member 301 is substantially the same as, for example, the length L1.

As shown in FIG. 3 and FIG. 5A, a space portion 303 is formed between one cylinder-side protrusion portion 341c and the other cylinder-side protrusion portion 341c in the circumferential direction of the cylinder member 301. The space portion 303 has substantially the same shape as the held portion 41c. The space portion 303 is in communication with the inside of the cylinder member 301 in the axial direction of the cylinder member 301.

As shown in FIG. 5A, the length of the space portion 303 in the circumferential direction of the cylinder member 301 is W3. This length W3 represents the distance between one cylinder-side protrusion portion 341c and the other cylinder-side protrusion portion 341c in the circumferential direction of the cylinder member 301. The length W3 is greater than the length L1, and the cylinder-side protrusion portions 341c are provided apart from each other in the circumferential direction of the cylinder member 301 so that the held portion 41c is inserted into the cylinder member 301 via the space portion 303 in the axial direction of the cylinder member 301.

That is, as shown in FIG. 5A, an inside diameter D2 of the one end portion 41a is substantially the same as the length L2 in the diametrical direction of the cylinder member 301. This inside diameter D2 represents the length from one space portion 303 to the other space portion 303.

As shown in FIG. 5A and FIG. 5B, a space portion 305 is formed between one cylinder-side protrusion portion 341c and the other cylinder-side protrusion portion 341c in the circumferential direction of the cylinder member 301. The space portion 305 is in communication with the inside of the cylinder member 301 in the axial direction of the cylinder member 301. The space portion 305 is in communication with the space portion 303 in the diametrical direction of the cylinder member 301. The space portion 305 is held between the space portions 303.

The space portion 305 has substantially the same shape as the one end portion 41a of the vibrator 41. The length of the space portion 305 is D3 in the diametrical direction of the cylinder member 301. This length D3 represents the distance between one cylinder-side protrusion portion 341c and the other cylinder-side protrusion portion 341c in the diametrical direction of the cylinder member 301. The cylinder-side protrusion portions 341c are provided apart from each other in the diametrical direction of the cylinder member 301 so that the length D3 is substantially the same as the diameter D1, the one end portion 41a of the vibrator 41 is inserted into the cylinder member 301 via the space portion 305 in the axial direction of the cylinder member 301.

As shown in FIG. 3, FIG. 5A, and FIG. 5B, the cylinder-side one end portion 341a has an end face 341b provided at the extreme end in the axial direction of the cylinder member 301. As shown in FIG. 3, FIG. 5A, and FIG. 5B, the cylinder-side protrusions 341c has an end face 341d provided at the extreme end in the axial direction of the cylinder member 301. As shown in FIG. 3, FIG. 5A, and FIG. 5B, the end face 341b and the end face 341d are formed in a plane that intersects at right angles with the axial direction of the cylinder member 301, and are flush. The end face 341b and the end face 341d are connected to each other, and are planes.

When the one end portion 51a of the probe 51 is inserted into the cylinder member 301 from the cylinder-side other end portion 351a as shown in FIG. 7A so that the probe-side connection portion 51g is connected to the cylinder-side connection portion 351g as shown in FIG. 7B, the cylinder-side protrusion portion 341c is provided at a distance equal to or more than the width W1 of the held portion 41c from the end face 511a in the axial direction of the cylinder member 301 as shown in FIG. 7B. At the same time, the cylinder-side protrusion portion 341c is provided relative to the end face 511a within the sum of the width W1 and the width W2 in the axial direction of the cylinder member 301. That is, as shown in FIG. 7B, W1<W4<W1+W2, wherein W4 is the distance between the cylinder-side protrusion portion 341c and the end face 511a in the axial direction of the cylinder member 301.

The cylinder-side protrusion portion 341c is also provided so that the held portion 41c is provided between the end face 511a of the one end portion 51a and a plane where the cylinder-side protrusion portion 341c is provided as shown in FIG. 7E in the axial direction of the cylinder member 301 when the one end portion 41a of the vibrator 41 is inserted into the cylinder member 301 from the cylinder-side one end portion 341a as shown in FIG. 7C. The plane where the cylinder-side protrusion portion 341c is provided represents a plane in the diametrical direction of the cylinder member 301.

The cylinder-side protrusion portion 341c is also provided so that at least part of the abutment portion 41e is provided collinearly with the cylinder-side protrusion portion 341c in the circumferential direction of the cylinder member 301 as shown in FIG. 7D and FIG. 7E when the one end portion 41a of the vibrator 41 is inserted into the cylinder member 301 from the cylinder-side one end portion 341a as shown in FIG. 7C.

The insertion of the one end portion 41a of the vibrator 41 into the cylinder member 301 from the cylinder-side one end portion 341a represents that the held portion 41c is inserted into the cylinder member 301 via the space portion 303 in the axial direction of the cylinder member 301, and at the same time, the one end portion 41a of the vibrator 41 is inserted into the cylinder member 301 via the space portion 305 in the axial direction of the cylinder member 301, as shown in FIG. 3.

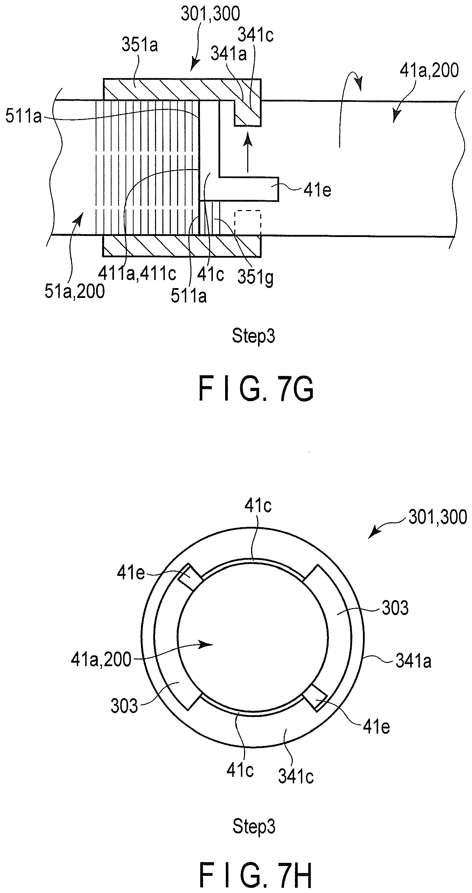

The cylinder-side protrusion portion 341c is also provided so that the abutment portion 41e comes into abutment with the cylinder-side protrusion portion 341c in the circumferential direction of the cylinder member 301 as shown in FIG. 7H and FIG. 7I when the one end portion 41a of the vibrator 41 is rotated relative to the cylinder member 301 around the axis of the cylinder member 301 as shown in FIG. 7F and FIG. 7G after the insertion of the one end portion 41a of the vibrator 41 into the cylinder member 301 from the cylinder-side one end portion 341a as shown in FIG. 7C. The cylinder-side protrusion portion 341c regulates the rotation of the one end portion 41a of the vibrator 41 relative to the cylinder member 301 when the abutment portion 41e comes into abutment with the cylinder-side protrusion portion 341c.

The cylinder-side protrusion portion 341c is also provided so that the held portion 41c is provided between the end face 511a and the cylinder-side protrusion portion 341c in the axial direction of the cylinder member 301 while the abutment portion 41e is in abutment with the cylinder-side protrusion portion 341c as shown in FIG. 7I.

The cylinder-side protrusion portion 341c is also provided so that the held portion 41c is held between the end face 511a and the cylinder-side protrusion portion 341c as shown in FIG. 7J and FIG. 7K when at least one of the one end portion 41a of the vibrator 41 and the one end portion 51a of the probe 51 is rotated relative to the cylinder member 301 around the axis of the cylinder member 301 and then further inserted into the cylinder member 301 so that the width W4 which is the distance between the end face 511a and the cylinder-side protrusion portion 341c is reduced to the width W1 as shown in FIG. 7J and FIG. 7K. Here, the held portion 41c is provided between the end face 511a and the cylinder-side protrusion portion 341c in the axial direction of the cylinder member 301 while the abutment portion 41e is in abutment with the cylinder-side protrusion portion 341c. As shown in FIG. 7J and FIG. 7K, the cylinder-side protrusion portion 341c is then provided so that the held portion 41c is in close contact with the end face 511a without any space therebetween and so that the held portion 41c is in close contact with the cylinder-side protrusion portion 341c without any space therebetween.

The cylinder-side connection portion 351g is, for example, a thread groove which engages with the probe-side connection portion 51g.

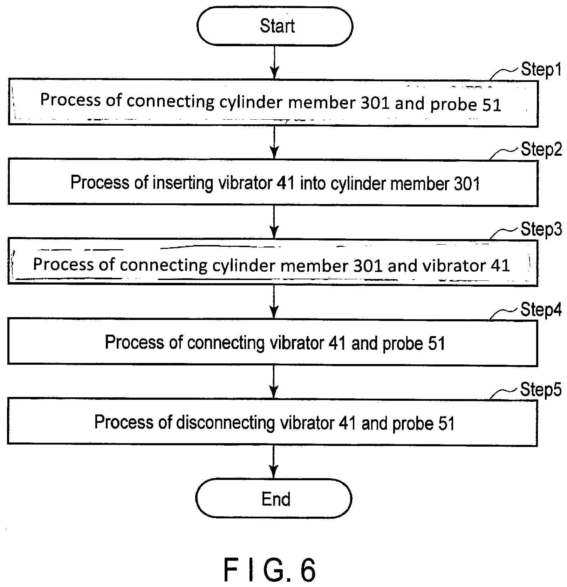

### [Production Method for Generating/Transmitting Unit 200 - FIG. 6]

### [Step 1 - Process of Temporarily Connecting Cylinder Member 301 and Probe 51 - FIG. 6, FIG. 7A, and FIG. 7B]

The probe-side connection portion 51g and the cylinder-side connection portion 351g are connected to each other in the axial direction of the cylinder member 301, and the one end portion 51a of the probe 51 is inserted into the cylinder member 301 from the cylinder-side other end portion 351a as shown in FIG. 7A so that the end face 511a is provided at a distance equal to or more than the width W1 from the cylinder-side protrusion portion 341c in the axial direction of the cylinder member 301 as shown in FIG. 7B. At the same time, the one end portion 51a is inserted into the cylinder member 301 if one of the one end portion 51a and the cylinder-side other end portion 351a is rotated halfway relative to the other.

The one end portion 51a is inserted into the cylinder-side other end portion 351a so that the abutment portion 41e will come into abutment with the cylinder-side protrusion portion 341c when at least part of the abutment portion 41e is provided collinearly with the cylinder-side protrusion portion 341c in the circumferential direction of the cylinder member 301 in later-described Step 2 (see FIG. 7E) and when the one end portion 41a of the vibrator 41 is rotated relative to the cylinder member 301 in Step 3 (see FIG. 7G and FIG. 7I).

### [Step 2 - Process of Inserting Vibrator 41 into Cylinder Member 301 - FIG. 6, FIG. 7C, FIG. 7D, and FIG. 7E]

The held portion 41c is inserted into the cylinder member 301 via the space portion 303 in the axial direction of the cylinder member 301 as shown in FIG. 7C and FIG. 7D so that the protrusion portion 51h is fitted into the depression portion 41h as shown in FIG. 7E. At the same time, the one end portion 41a of the vibrator 41 is inserted into the cylinder member 301 via the space portion 305 in the axial direction of the cylinder member 301 as shown in FIG. 7C and FIG. 7D.

Here, the one end portion 41a of the vibrator 41 is inserted into the cylinder member 301 from the cylinder-side one end portion 341a in the axial direction of the cylinder member 301 so that the held portion 41c is provided between the end face 511a and the plane where the cylinder-side protrusion portion 341c is provided in the axial direction of the cylinder member 301 as shown in FIG. 7E. At the same time, the one end portion 41a of the vibrator 41 is inserted into the cylinder member 301 from the cylinder-side one end portion 341a in the axial direction of the cylinder member 301 so that the at least part of the abutment portion 41e is provided collinearly with the cylinder-side protrusion portion 341c in the circumferential direction of the cylinder member 301 as shown in FIG. 7D and FIG. 7E.

Here, the one end portion 41a is also inserted into the cylinder member 301 from the cylinder-side one end portion 341a so that the end face 411a and the end face 411c will come into abutment with the end face 511a as shown in FIG. 7E.

### [Step 3 - Process of Temporarily Connecting Cylinder Member 301 and Vibrator 41 - FIG. 6, FIG. 7F, FIG. 7G, FIG. 7H, and FIG. 7I]

The one end portion 41a of the vibrator 41 is rotated halfway relative to the cylinder member 301 around the axis of the cylinder member 301 as shown in FIG. 7F and FIG. 7G so that the abutment portion 41e will come into abutment with the cylinder-side protrusion portion 341c in the circumferential direction of the cylinder member 301 as shown in FIG. 7H and FIG. 7I and in this abutment condition, the held portion 41c is provided between the end face 511a and the cylinder-side protrusion portion 341c in the axial direction of the cylinder member 301 as shown in FIG. 7H and FIG. 7I.

### [Step 4 - Process of Connecting Vibrator 41 and Probe 51 - FIG. 6, FIG. 7J, and FIG. 7K]

At least one of the one end portion 41a of the vibrator 41 and the one end portion 51a of the probe 51 is rotated around the axis of the cylinder member 301 and further inserted into the cylinder member 301 so that the width W4 shown in FIG. 7B which is the distance between the end face 511a and the cylinder-side protrusion portion 341c is reduced to the width W1 shown in FIG. 7J and so that the held portion 41c is held between the end face 511a and the cylinder-side protrusion portion 341c in the axial direction of the cylinder member 301 as shown in FIG. 7J and FIG. 7K. FIG. 7K is a diagram showing the condition in FIG. 7J seen from another position.

Here, for example, the one end portion 41a is rotated halfway clockwise relative to the cylinder member 301, and the one end portion 51a is rotated halfway counterclockwise relative to the cylinder member 301.

As a result, from the condition in Step 1, the probe-side connection portion 51g and the cylinder-side connection portion 351g further engage with each other, and the one end portion 51a is further inserted into the cylinder member 301 from the cylinder-side other end portion 351a as shown in FIG. 7J and FIG. 7K.

When the held portion 41c is held between the end face 511a and the cylinder-side protrusion portion 341c as shown in FIG. 7K and FIG. 7J, the held portion 41c is in close contact with the end face 511a without any space therebetween, and in close contact with the cylinder-side protrusion portion 341c without any space therebetween. Thus, the vibrator 41 and the probe 51 are firmly connected to each other without being out of alignment in the axial direction and the circumferential direction.

As shown in FIG. 7K and FIG. 7J, since the abutment portion 41e comes into abutment with the cylinder-side protrusion portion 341c, the vibrator 41 and the probe 51 are more firmly connected to each other without being out of alignment in the circumferential direction.

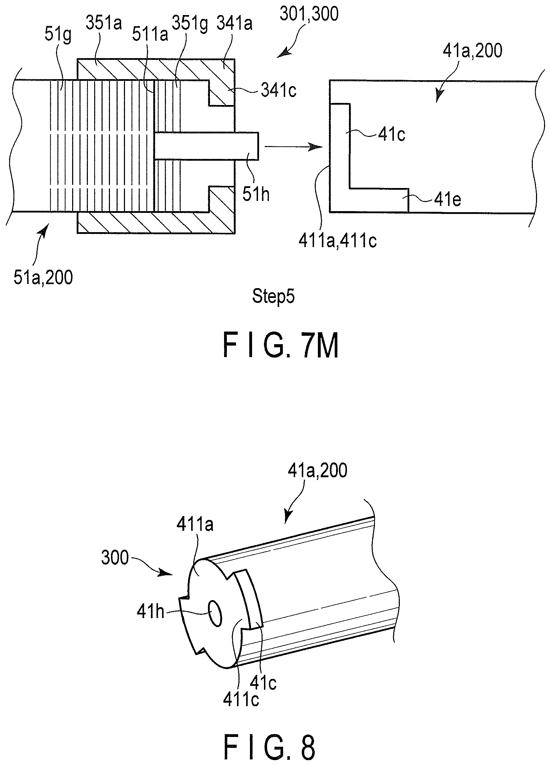

### [Step 5 - Process of Disconnecting Vibrator 41 and Probe 51 - FIG. 6, FIG. 7L, and FIG. 7M]

From the condition in Step 4, for example, the one end portion 51a is rotated halfway clockwise relative to the cylinder member 301 so that the held portion 41c will be separated from the cylinder-side protrusion portion 341c and the held portion 41c will be released from the condition in which the held portion 41c is held between the end face 511a and the cylinder-side protrusion portion 341c as shown in FIG. 7L.

The one end portion 41a of the vibrator 41 is then rotated, for example, halfway counterclockwise relative to the cylinder member 301 so that the abutment portion 41e will be separated from the cylinder-side protrusion portion 341c in the circumferential direction of the cylinder member 301 and so that the held portion 41c will be provided in the space portion 303 in the axial direction of the cylinder member 301 as shown in FIG. 7L.

As shown in FIG. 7M, the one end portion 41a of the vibrator 41 is then removed from the cylinder member 301 in the axial direction of the cylinder member 301.

### [Advantageous Effects in Connection Processes (Steps 1, 2, 3, and 4)]

According to the present embodiment, the vibrator 41 and the probe 51 are connected to each other by the cylinder member 301 as described above.

However, according to the present embodiment, in the production method for the generating/transmitting unit 200, after the vibrator 41 and the probe 51 are connected to each other, it is not necessary that the probe 51 be inserted into the cylinder member 301 from the other end portion 51b of the probe 51, that the cylinder member 301 slide on the probe 51 toward the one end portion 51a from the other end portion 51b of the probe 51, that the cylinder member 301 be rotated more than once, and that the cylinder member 301 fasten the connection part between the vibrator 41 and the probe 51 by rotation.

According to the present embodiment, the vibrator 41 is temporarily connected to the cylinder member 301 as shown in Steps 2 and 3 while the cylinder member 301 is temporarily connected to the one end portion 51a of the probe 51 as shown in Step 1. Finally, as shown in Step 4, the vibrator 41 and the probe 51 then rotate halfway relative to each other, and the held portion 41c is held by the end face 511a and the cylinder-side protrusion portion 341c, so that the vibrator 41 and the probe 51 are connected to each other.

Thus, according to the present embodiment, the number of rotations of the cylinder member 301 can be reduced, and the connecting operation can be performed without any trouble.

According to the present embodiment, since the end face 511a and the cylinder-side protrusion portion 341c hold the held portion 41c in between, the vibrator 41 and the probe 51 can be firmly connected to each other without being out of alignment in the axial direction and the circumferential direction.

According to the present embodiment, since the abutment portion 41e comes into abutment with the cylinder-side protrusion portion 341c, the vibrator 41 and the probe 51 can be more firmly connected to each other without being out of alignment in the circumferential direction, and the rotation of the vibrator 41 can be regulated.

According to the present embodiment, since the vibrator 41 and the probe 51 can be firmly connected to each other, ultrasonic vibration can be fully transmitted to the probe 51 from the vibrator 41.

According to the present embodiment, in Step 2, since the protrusion portion 51h is fitted into the depression portion 41h, the vibrator 41 can be easily positioned relative to the probe 51. Moreover, according to the present embodiment, since the space portion 303 has substantially the same shape as the held portion 41c, and the space portion 305 has substantially the same shape as the one end portion 41a, vibrator 41 can be easily positioned relative to the probe 51.

According to the present embodiment, a manufacturer can perform the process in Step 1, for example, at a manufacturing factory, and the user who uses the treatment apparatus 20 can perform the processes in Steps 2, 3, and 4. Therefore, according to the present embodiment, it is possible to save the user the trouble and improve usability.

According to the present embodiment, since, for example, the cylinder member 301 does not need to slide on the probe 51 during the connecting operation, it is possible to prevent the cylinder member 301 from being worn by sliding.

According to the present embodiment, the held portion 41c and the abutment portion 41e are provided in the one end portion 41a of the vibrator 41, and the end face 411a and the end face 411c are flush, so that the cylinder member 301 can be reduced in length.

According to the present embodiment, since the end face 511a is substantially the same size as the end face 411a, ultrasonic vibration can be fully transmitted to the probe 51 from the vibrator 41.

### [Advantageous Effects in Disconnection Process (Step 5)]

According to the present embodiment, the cylinder member 301 does not need to be separated from the vibrator 41 and the probe 51 as shown in Step 5. Thus, according to the present embodiment, in the disconnecting operation, the number of rotations of the cylinder member 301 can be reduced, and the disconnecting operation can be performed without any trouble.

According to the present embodiment, in the disconnecting operation, for example, the cylinder member 301 does not need to slide on the probe 51 toward the other end portion 51b from the one end portion 51a of the probe 51. Therefore, according to the present embodiment, it is possible to prevent the cylinder member 301 from being worn by sliding.

According to the present embodiment, the flow returns to Step 2 after Step 5, so that the probe 51 can be quickly replaced.

According to the present embodiment, the vibrator 41 has the held portion 41c and the abutment portion 41e, and the probe 51 has the probe-side connection portion 51g. However, the present invention does not need to be limited to this. It is only necessary that one of the vibrator 41 and the probe 51 have either the held portion 41c and the abutment portion 41e or the probe-side connection portion 51g, and that the other of the vibrator 41 and the probe 51 have either the held portion 41c and the abutment portion 41e or the probe-side connection portion 51g. Accordingly, the cylinder member 301 has only to have the cylinder-side protrusion portion 341c on the side of the held portion 41c and the abutment portion 41e, and have the cylinder-side connection portion 351g on the side of the probe-side connection portion 51g.

According to the present embodiment, the abutment portion 41e is provided along the axial direction of the one end portion 41a, and connected to the held portion 41c. However, it is only necessary that the abutment portion 41e can come into abutment with the cylinder-side protrusion portion 341c in Step 3. Thus, the abutment portion 41e has only to be provided to come into abutment with the cylinder-side protrusion portion 341c in the circumferential direction of the cylinder member 301 when the held portion 41c is held by the end face 511a and the cylinder-side protrusion portion 341c. Therefore, the abutment portion 41e is not particularly limited in its location, shape, and size.

### [Modifications]

According to the present embodiment, since the held portion 41c is held by the end face 511a and the cylinder-side protrusion portion 341c, the vibrator 41 and the probe 51 are firmly connected to each other without being out of alignment in the axial direction and the circumferential direction. Therefore, as shown in FIG. 8, the held portion 41c alone has only to be provided, and the abutment portion 41e does not always need to be provided. In this case, for example, in Step 4, since the cylinder member 301 or the probe 51, for example, is rotated, the held portion 41c is held by the end face 511a and the cylinder-side protrusion portion 341c.

When, for example, the held portion 41c alone is provided, the protrusion portion 51h and the depression portion 41h do not need to be columnar. In this case, the protrusion portion 51h and the depression portion 41h may have a shape such as a rectangular shape that prevents the vibrator 41 from being rotated relative to the probe 51. In this case, for example, in Step 4, the cylinder member 301, for example, is rotated, so that the held portion 41c is held by the end face 511a and the cylinder-side protrusion portion 341c. In this case, according to the present embodiment, the vibrator 41 and the probe 51 can be positioned in the circumferential direction, and can be firmly connected to each other without being out of alignment in the circumferential direction.

### [Second Embodiment]

### [Differences between Second Embodiment and First Embodiment]

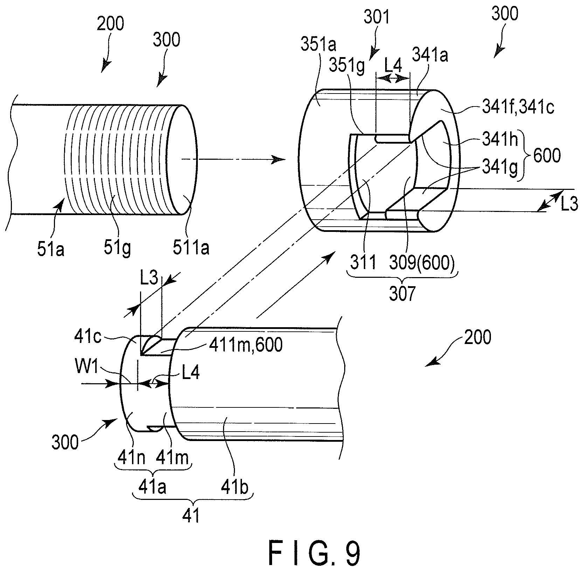

According to the first embodiment, the one end portion 41a of the vibrator 41 is inserted into the cylinder member 301 in the axial direction of the cylinder member 301, as shown in FIG. 3. However, the one end portion 41a of the vibrator 41 according to the present embodiment slides on to the cylinder member 301 in the diametrical direction of the cylinder member 301 and is thereby inserted into the cylinder member 301, as shown in FIG. 9.

### [Configuration]

### [Vibrator 41]

As shown in FIG. 9, the vibrator 41 includes the columnar one end portion 41a, and the columnar other end portion 41b which is provided coaxially with the one end portion 41a and which is thicker than the one end portion 41a.

The one end portion 41a is formed by a cutout end portion 41m connected to the other end portion 41b, and a columnar edge end portion 41n which is provided coaxially with the cutout end portion 41m and which is connected to the cutout end portion 41m.

The cutout end portion 41m has the shape of a column that is partly cut out. The cutout end portion 41m has, for example, a rectangular planar portion 411m formed by being cut out. The planar portion 411m is depressed from the outer circumferential surface of the cutout end portion 41m toward the central axis of the one end portion 41a. The planar portion 411m has a length L3 in the diametrical direction of the one end portion 41a which intersects at right angles with the depression direction, and has a width L4 in the axial direction of the one end portion 41a. The planar portion 411m is connected to the circumferential surface of the cutout end portion 41m. Two planar portions 411m, for example, are provided, and are provided 180 degrees apart from each other in the circumferential direction.

The cutout end portion 41m has a section formed along a sliding direction that intersects at right angles with the plane direction of the planar portion 411m. This section is formed by the planar portions 411m as an elliptic shape having two straight lines provided parallel to each other. More specifically, the section is formed by two straight lines parallel to each other, and two arcs which form part of a circle and which are connected to the ends of the straight lines.

The planar portion 411m functions as a prevention mechanism 600 which is provided on the outer circumferential surface of the one end portion 41a of the vibrator 41 and which prevents the one end portion 41a of the vibrator 41 inserted in the cylinder-side one end portion 341a from being rotated relative to the cylinder member 301 around the axis of the cylinder member 301.

The edge end portion 41n has a columnar shape. The edge end portion 41n has the held portion 41c. This held portion 41c is a protrusion portion formed by part of the edge end portion 41n diametrically protruding relative to the planar portion 411m. The length of the edge end portion 41n including the held portion 41c is W1.

### [Connection Mechanism 300 provided in Vibrator 41]

As shown in FIG. 9, the connection mechanism 300 includes the cutout end portion 41m and the edge end portion 41n that constitute the one end portion 41a of the vibrator 41, and the held portion 41c formed in the edge end portion 41n.

### [Connection Mechanism 300 provided in Probe 51]

As shown in FIG. 9, the protrusion portion 51h is omitted. In other respects, the connection mechanism 300 provided in the probe 51 is similar in configuration to that according to the first embodiment.

### [Cylinder Member 301]

As shown in FIG. 9, the cylinder member 301 has an opening portion 307 which is provided in the circumferential surface of the cylinder-side one end portion 341a of the cylinder member 301 and which is in communication with the inside the cylinder member 301 and the prevention mechanism 600 provided on the inner circumferential surface of the cylinder-side one end portion 341a. The opening portion 307 is provided so that the one end portion 41a of the vibrator 41 slides on the cylinder member 301 in the diametrical direction of the cylinder member 301 and is thereby inserted into the cylinder member 301 and provided in the cylinder member 301.

As shown in FIG. 9, the opening portion 307 is formed by a cutout opening portion 309 in which the cutout end portion 41m is inserted and provided in the cylinder member 301 in the diametrical direction of the cylinder member 301, and an edge end opening portion 311 in which the edge end portion 41n is inserted and provided in the cylinder member 301 in the diametrical direction of the cylinder member 301.

As shown in FIG. 9, the cutout opening portion 309 is regulated by protruding toward the central axis of the cylinder member 301 so that an edge portion 341f of the cylinder-side one end portion 341a is substantially U-shaped and so that the inner circumferential surface of the edge portion 341f will come into abutment with the held portion 41c formed in the planar portion 411m and the cutout end portion 41m. In other words, the inner circumferential surface of the edge portion 341f has two planar portions 341g which are substantially the same size as the planar portion 411m, and a circumferential surface 341h which is substantially the same size as part of the circumferential surface of the cutout end portion 41m. The cutout opening portion 309 is then regulated by the planar portions 341g and the circumferential surface 341h.

The cutout opening portion 309 is substantially the same size as the cutout end portion 41m, and positions the cutout end portion 41m.

The protruding amount of the edge portion 341f is substantially the same as the depressing amount of the planar portion 411m and the protruding amount of the held portion 41c.

In the axial direction of the cylinder member 301, the length of the edge portion 341f is substantially the same as the length L4 of the planar portion 411m.

In the diametrical direction of the cylinder member 301, the length of the planar portion 341g is substantially the same as the length L3 of the planar portion 411m.

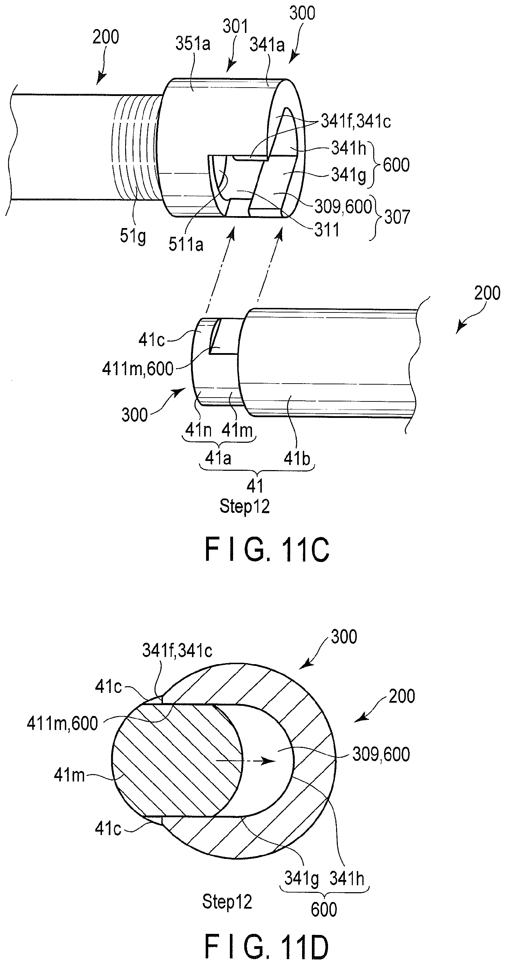

As shown in FIG. 11C and FIG. 11D, when the cutout end portion 41m is inserted into the cutout opening portion 309 in the diametrical direction of the cylinder member 301, the planar portion 411m slides on the planar portion 341g, and the circumferential surface of the cutout end portion 41m comes into abutment with the circumferential surface 341h.

As shown in FIG. 11C and FIG. 11D, the planar portion 341g comes into abutment with the planar portion 411m, and thereby prevents the one end portion 41a from being rotated relative to the cylinder member 301. Thus, the planar portion 341g, the circumferential surface 341h, and the cutout opening portion 309 function as the prevention mechanism 600 which is provided on the inner circumferential surface of the cylinder-side one end portion 341a and which prevents the one end portion 41a of the vibrator 41 inserted in the cylinder-side one end portion 341a from being rotated relative to the cylinder member 301 around the axis of the cylinder member 301.

As shown in FIG. 9 and FIG. 11D, the protruding edge portion 341f functions as the cylinder-side protrusion portion 341c which comes into abutment with the held portion 41c when the edge end portion 41n is inserted and provided in the cylinder member 301 via the edge end opening portion 311 in the diametrical direction of the cylinder member 301. The cylinder-side protrusion portion 341c comes into abutment with the held portion 41c, and thereby prevents the one end portion 41a from being removed from the cylinder-side one end portion 341a of the cylinder member 301.

The edge end opening portion 311 is substantially the same size as the edge end portion 41n, and positions the edge end portion 41n.

Thus, the opening portion 307 is provided so that the one end portion 41a of the vibrator 41 slides on the cylinder member 301 via the opening portion 307 in the diametrical direction of the cylinder member 301 and is thereby inserted into the cylinder member 301, so that the inserted held portion 41c is provided between the end face 511a and the cylinder-side protrusion portion 341c in the axial direction of the cylinder member 301, and so that the rotation of the inserted one end portion 41a of the vibrator 41 relative to the cylinder member 301 around the axis of the cylinder member 301 is prevented by the prevention mechanism 600.

Thus, since the rotation of the one end portion 41a of the vibrator 41 is prevented, the cylinder-side protrusion portion 341c is provided so that the held portion 41c is held by the end face 511a and the cylinder-side protrusion portion 341c when the one end portion 51a of the probe 51 is rotated relative to the cylinder member 301 around the axis of the cylinder member 301 and then further inserted into the cylinder member 301 so that the width W1 which is the distance between the end face 511a and the cylinder-side protrusion portion 341c is reduced.

### [Production Method for Generating/Transmitting Unit 200]

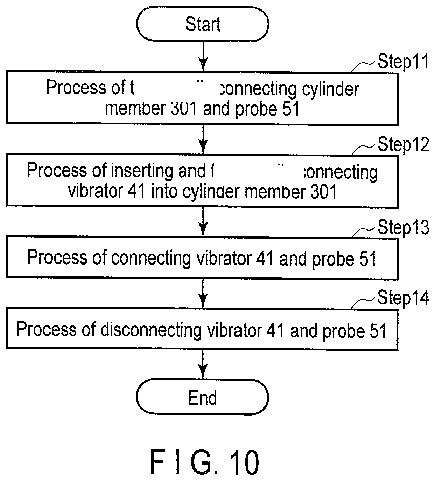

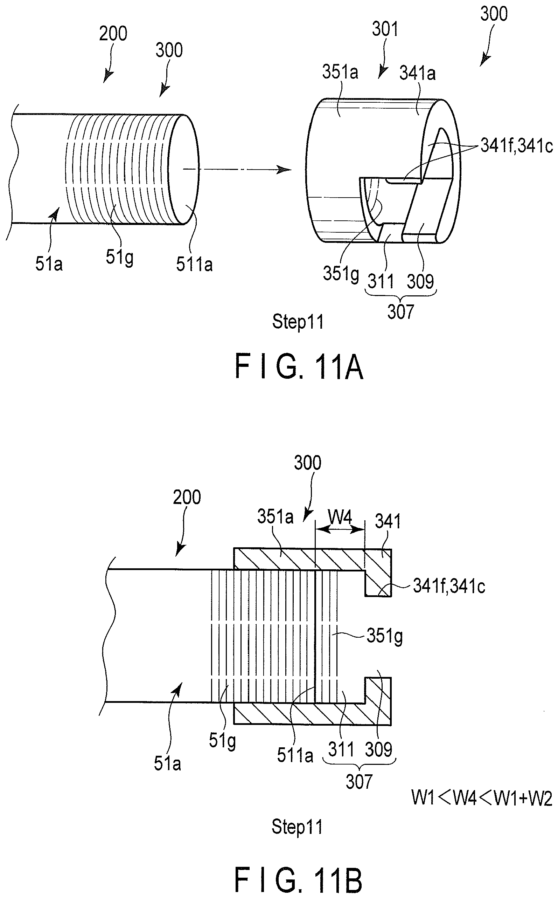

### [Step 11 - Process of Temporarily Connecting Cylinder Member 301 and Probe 51 - FIG. 10, FIG. 11A, and FIG. 11B]

Step 11 is substantially similar to Step 1.

The one end portion 51a of the probe 51 is inserted into the cylinder member 301 from the cylinder-side other end portion 351a as shown in FIG. 11A while the probe-side connection portion 51g and the cylinder-side connection portion 351g are connected to each other in the axial direction of the cylinder member 301 so that the end face 511a is provided at a distance equal to or more than the width W1 from the cylinder-side protrusion portion 341c functioning as the edge portion 341f in the axial direction of the cylinder member 301 as shown in FIG. 11B. Here, the one end portion 51a is inserted into the cylinder member 301 if one of the one end portion 51a and the cylinder-side other end portion 351a is, for example, rotated halfway relative to the other.

### [Step 12 - Process of Inserting and Temporarily Connecting Vibrator 41 into Cylinder Member 301 - FIG. 11C and FIG. 11D]

The one end portion 41a slides on the cylinder member 301 via the opening portion 307 in the diametrical direction of the cylinder member 301 and is thereby inserted into the cylinder member 301 as shown in FIG. 11C and FIG. 11D so that the inserted held portion 41c is provided between the end face 511a and the cylinder-side protrusion portion 341c in the axial direction of the cylinder member 301 and so that the rotation of the inserted one end portion 41a of the vibrator 41 relative to the cylinder member 301 around the axis of the cylinder member 301 is prevented by the prevention mechanism 600.

More specifically, the cutout end portion 41m is inserted from the cutout opening portion 309 and then provided in the cutout opening portion 309 as shown in FIG. 11C and FIG. 11D, and at the same time, the edge end portion 41n is inserted into the edge end opening portion 311 and provided in the edge end opening portion 311.

Here, as shown in FIG. 11D, the planar portion 411m of the cutout end portion 41m slides on the planar portion 341g, and the circumferential surface 341h comes into abutment with the circumferential surface of the cutout end portion 41m. As a result, the rotation of the one end portion 41a relative to the cylinder member 301 is prevented.

At the same time, the held portion 41c slides on the cylinder-side protrusion portion 341c functioning as the edge portion 341f. The removal of the one end portion 41a from the one end portion of the cylinder member 301 is prevented.

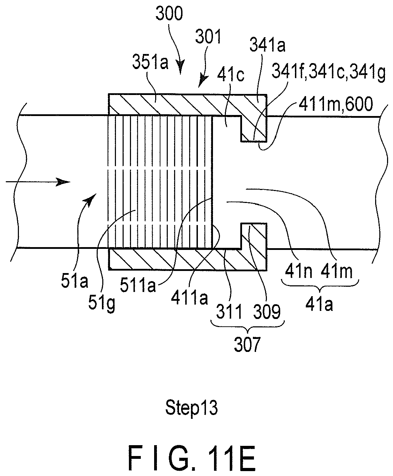

### [Step 13 - Process of Connecting Vibrator 41 and Probe 51 - FIG. 11E]

The one end portion 51a of the probe 51 is rotated around the axis of the cylinder member 301 and further inserted into the cylinder member 301 so that the width W4 which is the distance between the end face 511a and the cylinder-side protrusion portion 341c is reduced to the width W1 and so that the held portion 41c is held between the end face 511a and the cylinder-side protrusion portion 341c in the axial direction of the cylinder member 301, as shown in FIG. 11E. For example, the one end portion 51a of the probe 51 is rotated halfway counterclockwise relative to the cylinder member 301.

Here, from the condition in Step 11, the one end portion 51a of the probe 51 is further inserted into the cylinder-side other end portion 351a.

When the held portion 41c is held by the end face 511a and the cylinder-side protrusion portion 341c functioning as the edge portion 341f, the held portion 41c is in close contact with the end face 511a without any space therebetween, and in close contact with the cylinder-side protrusion portion 341c functioning as the edge portion 341f without any space therebetween. Thus, the vibrator 41 and the probe 51 are firmly connected to each other without being out of alignment in the axial direction and the circumferential direction.

Since the planar portion 341g comes into abutment with the planar portion 411m, the vibrator 41 and the probe 51 are more firmly connected to each other without being out of alignment in the circumferential direction.

### [Step 14 - Process of Disconnecting Vibrator 41 and Probe 51]

From the condition in Step 13 shown in FIG. 11E, the one end portion 51a of the probe 51 is rotated, for example, halfway clockwise relative to the cylinder member 301 so that the end face 511a will be separated from the held portion 41c and the held portion 41c will be released from the condition in which the held portion 41c is held between the end face 511a and the cylinder-side protrusion portion 341c functioning as the edge portion 341f.

The one end portion 41a of the vibrator 41 then slides on the cylinder member 301 via the opening portion 307 in the diametrical direction of the cylinder member 301 and is thereby removed from the cylinder member 301 as shown in FIG. 11C and FIG. 11D.

### [Advantageous Effects]

Thus, according to the present embodiment, the one end portion 41a of the vibrator 41 slides on the cylinder member 301 via the opening portion 307 in the diametrical direction of the cylinder member 301, so that advantageous effects similar to those according to the first embodiment can be obtained even if the one end portion 41a of the vibrator 41 is inserted into or removed from the cylinder member 301.

According to the first and second embodiments, the vibrator 41 which is one part constituting the generating/transmitting unit 200 and the probe 51 which is the other part constituting the generating/transmitting unit 200 have been described by way of example in the operation of connecting and disconnecting the parts constituting the generating/transmitting unit 200. However, the present invention does not need to be limited to this. For example, when the probe 51 is formed by more than one component, the first and second embodiments are applicable to both the connection and disconnection of one component and the other component.

Additional advantages and modifications will readily occur to those skilled in the art. Therefore, the invention in its broader aspects is not limited to the specific details and representative embodiments shown and described herein. Accordingly, various modifications may be made without departing from the scope of the general inventive concept as defined by the appended claims and their equivalents.

## Claims

1. A connection mechanism (300) for an ultrasonic-vibration generating/transmitting unit, the connection mechanism (300) comprising:
a first member (51) which has an end face (511a) at one end portion (51a) and which is provided with a first connection portion (51g) at the one end portion (51a) and which is configured to transmit ultrasonic vibration;
a second member (41) which is provided with a diametrically protruding held portion (41c) at one end portion (41a), which is configured to transmit the ultrasonic vibration, the length (L1) of the held portion (41c) in the circumferential direction of the one end portion (41a) being a prescribed length; and
a cylindrical third member (301) which has one end portion (341a) and another end portion (351a) and which connects the first member (51) and the second member (41) to each other,
the third member (301) comprising a protrusion portion (341c) which protrudes inward from an inner circumferential surface at the one end portion (341a) of the third member (301), an opening portion (D2, 307) which is provided at the one end portion (341a) of the third member (301), and the opening portion (D2, 307) being longer than the prescribed length of the second member (41) so that the held portion (41c) is able to be inserted into or removed from the opening portion (D2, 307), and
a second connection portion (351g) which is provided on the inner circumferential surface of the other end portion (351a) of the third member (301) and which is connected to the first connection portion (51g)
**characterized in that** the protrusion portion (341c) forms a first space portion (W4) between the end face (511a) of the first member (51) and the protrusion portion (341c) while the first member (51) is inserted from the other end portion (351a) of the third member (301),
and the first connection portion (51g) is movable relative to the third member (301) in the axial direction of the third member (301) to adjust the length of the first space portion (W4) formed between the end face (511a) of the first member (51) and the protrusion portion (341c), wherein,
with the held portion (41c) of the second member (41) inserted from the opening portion (D2, 307) of the third member (301), and disposed in the first space portion (W4) between the protrusion portion (341c) and the end face (511a) of the first member (51), the third member (301) moves the protrusion portion (341c) relative to the first member (51) so that the protrusion portion (341c) presses the held portion (41c) against the end face (511a) of the first member (51), and the third member (301) thereby holds the held portion (41c) between the end face (511a) and the protrusion portion (341c).

2. The connection mechanism (300) for the ultrasonic-vibration generating/transmitting unit according to claim 1, wherein the second member (41) comprises two held portions (41c) spaced apart from each other in the circumferential direction of the one end portion (41a) of the second member (41) and where a distance (L2) between the one and the other held portion (41c) in the diametrical direction of the one end portion (41a) of the second member (41) is the prescribed length.

3. The connection mechanism (300) for the ultrasonic-vibration generating/transmitting unit according to claim 1, wherein the third member (301) comprises two protrusion portions (341c) spaced apart from each other in a circumferential direction of the one end portion (341a) of the third member (301), and where the third member (301) forms a second space portion (303, 311) in between the one protrusion portion (341c) and the other protrusion portion (341c) in a circumferential direction of the one end portion (341a) of the third member (301).

4. The connection mechanism (300) for the ultrasonic-vibration generating/transmitting unit according to claim 3, wherein the second space portion (303, 311) is a shape so that the held portion (41c) is able to be inserted into or removed from the second space portion (303, 311).

5. The connection mechanism (300) for the ultrasonic-vibration generating/transmitting unit according to claim 1, wherein the second member (41) has a cutout portion (41m) which is formed at another end portion (41b) side of the second member (41) at the held portion (41c), the third member (301) has a cutout opening portion (309) which is formed at the protrusion portion (341c) and in which the cutout portion (41m) is configured to be inserted into, and the cutout portion (41m) is provided in the cutout opening portion (309), when the held portion (41c) is provided in the opening portion (307).

6. The connection mechanism (300) for the ultrasonic-vibration generating/transmitting unit according to claim 1, wherein the held portion (41c) has one end face (411c) and an abutment portion (41e), and brings the abutment portion (41e) into abutment with the protrusion portion (341c).

7. The connection mechanism (300) for the ultrasonic-vibration generating/transmitting unit according to claim 6, wherein the end face (511a) of the first member (51) is a first end face,
the second member (41) has, at the one end portion (41a), second end face (411a, 411c) which comes into abutment with the end face of the first member (51),
the second end face (411a, 411c) has at least the one end face (411c) of the held portion (41c), and
the third member (301) connects the end face of the first member (51) and the second end face (411a, 411c) of the second member (41) to each other.

8. The connection mechanism (300) for the ultrasonic-vibration generating/transmitting unit according to claim 1, wherein when the one end portion (51a) of the first member (51) is inserted into the third member (301) from the other end portion (351a) of the third member (301) so that the first connection portion (51g) is connected to the second connection portion (351g), the protrusion portion (341c) is provided at a distance greater than a width W1 of the diametrically protruding held portion (41c) from the end face (511a) of the first member (51) in the axial direction of the third member (301).

9. The connection mechanism (300) for the ultrasonic-vibration generating/transmitting unit according to claim 8, further comprising an abutment portion (41e) which is provided on the outer circumferential surface of the one end portion (41a) of the second member (41)and which comes into abutment with the protrusion portion (341c) in the circumferential direction of the third member (301) when the held portion (41c) is held by the end face (511a) of the first member (51) and the protrusion portion (341c),
wherein when the one end portion (51a) of the first member (51) is inserted into the third member (301) from the other end portion (351a) of the third member (301) along the axial direction of the third member (301),
the held portion (41c) is provided between the end face (511a) of the first member (51) and a plane where the protrusion portion (341c) is provided in the axial direction of the third member (301), and
the abutment portion (41e) is provided collinearly with the protrusion portion in the circumferential direction of the third member(301).

10. The connection mechanism (300) for the ultrasonic-vibration generating/transmitting unit according to claim 8, further comprising a prevention mechanism (341g, 341h, 309, 411m, 600) which is provided on an inner circumferential surface of the one end portion (341a) of the third member (301) and an outer circumferential surface of the one end portion (41a) of the second member (41) and which prevents the one end portion (41a) of the second member (41) when inserted in the one end portion (341a) of the third member (301) from being rotated relative to the third member (301) around the axis of the third member (301), wherein the opening portion (307) of the third member (301) is provided in the circumferential surface of the one end portion (341a) of the third member (301) and is in communication with the inside of the third member (301) and the prevention mechanism (341g, 341h, 309, 411m, 600) is provided on the inner circumferential surface of the third member (301), and the opening portion (307) is configured so that the one end portion (41a) of the second member (41) slides on the third member (301) via the opening portion (307) in the direction orthogonal to the longitudinal axis of the third member (301) and is thereby inserted into the third member (301), so that the inserted held portion (41c) is provided between the end face (511a) of the first member (51) and the protrusion portion (341c) in the axial direction of the third member (301), and so that the rotation of the inserted one end portion (51a) of the first member (51) relative to the third member (301) around the axis of the third member (301) is prevented by the prevention mechanism (341g, 341h, 309, 411m, 600).

11. A production method for an ultrasonic-vibration generating/transmitting unit, wherein there are provided:
a connection mechanism (300) according to claim 1, the production method comprising a connection process, the connection process comprising inserting the one end portion (41a) of the second member (41) into the third member (301) from the one end portion (341a) of the third member (301) so that the held portion (41c) is held by the end face (511a) of the first member (51) and the protrusion portion (341c) in the axial direction of the third member (301), inserting the one end portion (51a) of the first member (51) into the third member (301) from the other end portion (351a) of the third member (301) so that the second connection portion (351g) is connected to the first connection portion (51g), and thereby connecting the one end portion (51a) of the first member (51) and the one end portion (41a) of the second member (41) to each other.

12. The production method for the ultrasonic-vibration generating/transmitting unit according to claim 11, wherein the connection mechanism (300) further comprises the features of claim 2.

13. The production method for the ultrasonic-vibration generating/transmitting unit according to claim 11, wherein the connection mechanism (300) further comprises the features of claim 3.

14. The production method for the ultrasonic-vibration generating/transmitting unit according to claim 11, wherein there the connection mechanism (300) further comprises the features of claim 5.

15. The production method for the ultrasonic-vibration generating/transmitting unit according to claim 11, wherein the end face (511a) of the first member (51) is a first end face, and
the second member (41) has, at the one end portion (41a), a second end face (411a, 411c) which comes into abutment with the end face of the first member (51),
the production method further comprising a process of connecting the first member (51) and the third member (301) wherein the one end portion (51a) of the first member (51) is inserted into the third member (301) from the other end portion (351a) of the third member (301) while the first connection portion (51g) is connected to the second connection portion (351g) so that the end face (511a) of the first member (51) is provided at a distance greater than a width W1 of the held portion (41c) from the protrusion portion (341c) in the axial direction of the third member (301).

## Patentansprüche

1. Verbindungsmechanismus (300) für eine Ultraschallschwingungserzeugungs-/Ultraschallschwingungsübertragungseinheit, wobei der Verbindungsmechanismus (300) umfasst:
ein erstes Element (51), das eine Endfläche (511a) an einem Endabschnitt (51a) aufweist und das mit einem ersten Verbindungsabschnitt (51g) an dem einem Endabschnitt (51a) versehen ist und das so konfiguriert ist, dass es Ultraschallschwingung überträgt;
ein zweites Element (41), das mit einem diametrisch vorspringenden gehaltenen Abschnitt (41c) an einem Endabschnitt (41a) versehen ist, das so konfiguriert ist, dass es die Ultraschallschwingung überträgt, wobei die Länge (L1) des gehaltenen Abschnitts (41c) in Umfangsrichtung des einen Endabschnitts (41a) eine vorgeschriebene Länge ist; und
ein zylindrisches drittes Element (301), das einen Endabschnitt (341a) und einen weiteren Endabschnitt (351a) aufweist und das erste Element (51) und das zweite Element (41) miteinander verbindet,
wobei das dritte Element (301) einen Vorsprungsabschnitt (341c) umfasst, der von einer Innenumfangsfläche an dem einen Endabschnitt (341a) des dritten Elements (301) nach innen vorspringt,
einen Öffnungsabschnitt (D2, 307), der an dem einen Endabschnitt (341a) des dritten Elements (301) vorgesehen ist, und wobei der Öffnungsabschnitt (D2, 307) länger als die vorgeschriebene Länge des zweiten Elements (41) ist, so dass der gehaltene Abschnitt (41c) in der Lage ist, in den Öffnungsabschnitt (D2, 307) eingeführt oder aus diesem entfernt zu werden, und
einen zweiten Verbindungsabschnitt (351g), der auf der Innenumfangsfläche des anderen Endabschnitts (351a) des dritten Elements (301) vorgesehen ist und der mit dem ersten Verbindungsabschnitt (51g) verbunden ist,
**dadurch gekennzeichnet, dass**:
der Vorsprungsabschnitt (341c) einen ersten Raumabschnitt (W4) zwischen der Endfläche (511a) des ersten Elements (51) und dem Vorsprungsabschnitt (341c) bildet, während das erste Element (51) vom anderen Endabschnitt (351a) des dritten Elements (301) eingeführt ist,
und der erste Verbindungsabschnitt (51g) in Bezug auf das dritte Element (301) in axialer Richtung des dritten Elements (301) beweglich ist, um die Länge des ersten Raumabschnitts (W4) anzupassen, die zwischen der Endfläche (511a) des ersten Elements (51) und dem Vorsprungsabschnitt (341c) gebildet ist, wobei
wenn der gehaltene Abschnitt (41c) des zweiten Elements (41) vom Öffnungsabschnitt (D2, 307) des dritten Elements (301) eingeführt und im ersten Raumabschnitt (W4) zwischen dem Vorsprungsabschnitt (341c) und der Endfläche (511a) des ersten Elements (51) angeordnet ist, das dritte Element (301) den Vorsprungsabschnitt (341a) in Bezug auf das erste Element (51) bewegt, so dass der Vorsprungsabschnitt (341c) den gehaltenen Abschnitt (41c) gegen die Endfläche (511a) des ersten Elements (51) drückt und das dritte Element (301) somit den gehaltenen Abschnitt (41c) zwischen der Endfläche (511a) und dem Vorsprungsabschnitt (341c) hält.

2. Verbindungsmechanismus (300) für eine Ultraschallschwingungserzeugungs-/Ultraschallschwingungsübertragungseinheit nach Anspruch 1, wobei das zweite Element (41) zwei gehaltene Abschnitte (41c) umfasst, die voneinander im Umfangsrichtung des einen Endabschnitts (41a) des zweiten Elements (41) beabstandet sind, und wobei eine Distanz (L2) zwischen dem einen und dem anderen gehaltenen Abschnitt (41c) in diametrischer Richtung des einen Endabschnitts (41a) des zweiten Elements (41) die vorgeschriebene Länge ist.

3. Verbindungsmechanismus (300) für eine Ultraschallschwingungserzeugungs-/Ultraschallschwingungsübertragungseinheit nach Anspruch 1, wobei das dritte Element (301) zwei Vorsprungsabschnitte (341c) umfasst, die voneinander im Umfangsrichtung des einen Endabschnitts (341a) des dritten Elements (301) beabstandet sind, und wobei das dritte Element (301) einen zweiten Raumabschnitt (303, 311) zwischen dem einen Vorsprungsabschnitt (341c) und dem anderen Vorsprungsabschnitt (341c) in Umfangsrichtung des einen Endabschnitts (341a) des dritten Elements (301) bildet.

4. Verbindungsmechanismus (300) für eine Ultraschallschwingungserzeugungs-/Ultraschallschwingungsübertragungseinheit nach Anspruch 3, wobei der zweite Raumabschnitt (303, 311) eine solche Form hat, dass der gehaltene Abschnitt (41c) in der Lage ist, in den zweiten Raumabschnitt (303, 311) eingeführt oder aus diesem entfernt zu werden.

5. Verbindungsmechanismus (300) für eine Ultraschallschwingungserzeugungs-/Ultraschallschwingungsübertragungseinheit nach Anspruch 1, wobei das zweite Element (41) einen ausgeschnittenen Abschnitt (41m) aufweist, der an einer anderen Seite eines Endabschnitts (41b) des zweiten Elements (41) am gehaltenen Abschnitt (41c) gebildet ist,
das dritte Element (301) einen ausgeschnittenen Öffnungsabschnitt (309) aufweist, der am Vorsprungsabschnitt (341c) gebildet ist, und wobei der ausgeschnittene Abschnitt (41m) so konfiguriert ist, dass er in diesen eingeführt wird, und
der ausgeschnittene Abschnitt (41m) im ausgeschnittenen Öffnungsabschnitt (309) vorgesehen ist, wenn der gehaltene Abschnitt (41c) im Öffnungsabschnitt (307) vorgesehen ist.

6. Verbindungsmechanismus (300) für eine Ultraschallschwingungserzeugungs-/Ultraschallschwingungsübertragungseinheit nach Anspruch 1, wobei der gehaltene Abschnitt (41c) eine Endfläche (411c) und einen Anliegeabschnitt (41e) umfasst und den Anliegeabschnitt (41c) zum Anliegen am Vorsprungsabschnitt (341c) bringt.

7. Verbindungsmechanismus (300) für eine Ultraschallschwingungserzeugungs-/Ultraschallschwingungsübertragungseinheit nach Anspruch 6, wobei die Endfläche (511a) des ersten Elements (51) eine erste Endfläche ist,
wobei das zweite Element (41) an dem einen Endabschnitt (41a) eine zweite Endfläche (411a, 411c) aufweist, die an der Endfläche des ersten Elements (51) anliegt,
wobei die zweite Endfläche (411a, 411c) zumindest die eine Endfläche (411c) des gehaltenen Abschnitts (41c) aufweist, und
wobei das dritte Element (301) die Endfläche des ersten Elements (51) und die zweite Endfläche (411a, 411c) des zweiten Elements (41) miteinander verbindet.

8. Verbindungsmechanismus (300) für eine Ultraschallschwingungserzeugungs-/Ultraschallschwingungsübertragungseinheit nach Anspruch 1, wobei, wenn der eine Endabschnitt (51a) des ersten Elements (51) vom anderen Endabschnitt (351a) des dritten Elements (301) in das dritte Element (301) eingeführt ist, so dass der erste Verbindungsabschnitt (51g) mit dem zweiten Verbindungsabschnitt (351g) verbunden ist, der Vorsprungsabschnitt (341c) in einer Distanz vorgesehen ist, die größer als eine Breite W1 des diametrisch vorspringenden gehaltenen Abschnitts (41c) von der Endfläche (511a) des ersten Elements (51) in axialer Richtung des dritten Elements (301) ist.

9. Verbindungsmechanismus (300) für eine Ultraschallschwingungserzeugungs-/Ultraschallschwingungsübertragungseinheit nach Anspruch 8, der ferner einen Anliegeabschnitt (41e) umfasst, der auf der Außenumfangsfläche des einen Endabschnitts (41a) des zweiten Elements (41) vorgesehen ist und am Vorsprungsabschnitt (341c) in Umfangsrichtung des dritten Elements (301) anliegt, wenn der gehaltene Abschnitt (41c) von der Endfläche (511a) des ersten Elements (51) und dem Vorsprungsabschnitt (341c) gehalten wird,
wobei, wenn der eine Endabschnitt (51a) des ersten Elements (51) vom anderen Endabschnitt (351a) des dritten Elements (301) entlang der axialen Richtung des dritten Elements (301) in das dritte Element (301) eingeführt ist,
der gehaltene Abschnitt (41c) zwischen der Endfläche (511a) des ersten Elements (51) und einer Ebene vorgesehen ist, wobei der Vorsprungsabschnitt (341c) in axialer Richtung des dritten Elements (301) vorgesehen ist, und
der Anliegeabschnitt (41c) kolinear mit dem Vorsprungsabschnitt in Umfangsrichtung des dritten Elements (301) vorgesehen ist.

10. Verbindungsmechanismus (300) für eine Ultraschallschwingungserzeugungs-/Ultraschallschwingungsübertragungseinheit nach Anspruch 8, der ferner einen Verhinderungsmechanismus (341g, 341h, 309, 411m, 600) umfasst, der auf einer Innenumfangsfläche des Endabschnitts (341a) des dritten Elements (301) und einer Außenumfangsfläche des einen Endabschnitts (41a) des zweiten Elements (41) vorgesehen ist und verhindert, dass der eine Endabschnitt (41a) des zweiten Elements (41), wenn in den einen Endabschnitt (341a) des dritten Elements (301) eingeführt, in Bezug auf das dritte Element (301) um die Achse des dritten Elements (301) gedreht wird,
wobei der Öffnungsabschnitt (307) des dritten Elements (301) in der Umfangsfläche des einen Endabschnitts (341a) des dritten Elements (301) vorgesehen und mit dem Inneren des dritten Elements (301) in Kommunikation steht und wobei der Verhinderungsmechanismus (341g, 341h, 309, 411m, 600) auf der Innenumfangsfläche des dritten Elements (301) vorgesehen ist und wobei der Öffnungsabschnitt (307) so konfiguriert ist, dass der eine Endabschnitt (41a) des zweiten Elements (41) über den Öffnungsabschnitt (307) in der Richtung orthogonal zur Längsachse des dritten Elements (301) auf das dritte Element (301) gleitet und somit in das dritte Element (301) eingeführt wird, so dass der eingeführte gehaltene Abschnitt (41c) zwischen der Endfläche (511a) des ersten Elements (51) und dem Vorsprungsabschnitt (341c) in axialer Richtung des dritten Elements (301) vorgesehen ist und so dass die Drehung des eingeführten einen Endabschnitts (51a) des ersten Elements (51) in Bezug auf das dritte Element (301) um die Achse des dritten Elements (301) durch den Verhinderungsmechanismus (341g, 341h, 309, 411m, 600) verhindert wird.

11. Verfahren zum Herstellen einer Ultraschallschwingungserzeugungs-/Ultraschallschwingungsübertragungseinheit, wobei vorgesehen ist:
ein Verbindungsmechanismus (300) nach Anspruch 1,
wobei das Herstellungsverfahren einen Verbindungsprozess umfasst, wobei der Verbindungsprozess Einsetzen des einen Endabschnitts (41a) des zweiten Elements (41) von dem einen Endabschnitt (341a) des dritten Elements (301) in das dritte Element (301) umfasst, so dass der gehaltene Abschnitt (41c) von der Endfläche (511a) des ersten Elements (51) und dem Vorsprungsabschnitt (341c) in axialer Richtung des dritten Elements (301) gehalten wird, Einsetzen des einen Endabschnitts (51a) des ersten Elements (51) vom anderen Endabschnitt (351a) des dritten Elements (301) in das dritte Element (301), so dass der Verbindungsabschnitt (351g) mit dem ersten Verbindungsabschnitt (51g) verbunden ist, und somit Verbinden des einen Endabschnitts (51a) des ersten Elements (51) und des einen Endabschnitts (41a) des zweiten Elements (41) miteinander.

12. Verfahren zum Herstellen einer Ultraschallschwingungserzeugungs-/Ultraschallschwingungsübertragungseinheit nach Anspruch 11, wobei der Verbindungsmechanismus (300) ferner die Merkmale von Anspruch 2 umfasst.

13. Verfahren zum Herstellen einer Ultraschallschwingungserzeugungs-/Ultraschallschwingungsübertragungseinheit nach Anspruch 11, wobei der Verbindungsmechanismus (300) ferner die Merkmale von Anspruch 3 umfasst.

14. Verfahren zum Herstellen einer Ultraschallschwingungserzeugungs-/Ultraschallschwingungsübertragungseinheit nach Anspruch 11, wobei der Verbindungsmechanismus (300) ferner die Merkmale von Anspruch 5 umfasst.

15. Herstellungsverfahren für eine Ultraschallschwingungserzeugungs-/Ultraschallschwingungsübertragungseinheit nach Anspruch 11, wobei die Endfläche (511a) des ersten Elements (51) eine erste Endfläche ist, und
das zweite Element (41) an dem einen Endabschnitt (41a) eine zweite Endfläche (411a, 411c) aufweist, die an der Endfläche des ersten Elements (51) anliegt,
wobei das Herstellungsverfahren ferner einen Prozess zum Verbinden des ersten Elements (51) und des dritten Elements (301) umfasst, wobei der eine Endabschnitt (51a) des ersten Elements (51) vom anderen Endabschnitt (351a) des dritten Elements (301) in das dritte Element (301) eingeführt wird, während der erste Verbindungsabschnitt (51g) mit dem zweiten Verbindungsabschnitt (351g) verbunden ist, so dass die Endfläche (511a) des ersten Elements (51) in einer Distanz vorgesehen ist, die größer als eine Breite W1 des gehaltenen Abschnitts (41c) vom Vorsprungsabschnitt (341c) in axialer Richtung des dritten Elements (301) ist.

## Revendications

1. Mécanisme de connexion (300) pour une unité de génération/transmission de vibration ultrasonore, le mécanisme de connexion (300) comprenant :
un premier élément (51) qui a une face d'extrémité (511a) au niveau d'une première partie d'extrémité (51a) et qui comporte une première partie de connexion (51g) au niveau de la première partie d'extrémité (51a) et qui est configurée pour transmettre une vibration ultrasonore ;
un deuxième élément (41) qui comporte une partie maintenue faisant diamétralement saillie (41c) au niveau d'une première partie d'extrémité (41a), qui est configurée pour transmettre la vibration ultrasonore, la longueur (L1) de la partie maintenue (41c) dans la direction circonférentielle de la première partie d'extrémité (41a) étant une longueur prescrite ; et
un troisième élément cylindrique (301) qui a une première partie d'extrémité (341a) et une autre partie d'extrémité (351a) et qui relie le premier élément (51) et le deuxième élément (41) l'un à l'autre,
le troisième élément (301) comprenant une partie de saillie (341c) qui fait saillie vers l'intérieur à partir d'une surface circonférentielle interne au niveau de la première partie d'extrémité (341a) du troisième élément (301),
une partie d'ouverture (D2, 307) qui est disposée au niveau de la première partie d'extrémité (341a) du troisième élément (301), et la partie d'ouverture (D2, 307) étant plus longue que la longueur prescrite du deuxième élément (41) de telle sorte que la partie maintenue (41c) est apte à être insérée dans ou être retirée de la partie d'ouverture (D2, 307), et
une seconde partie de connexion (351g) qui est disposée sur la surface circonférentielle interne de l'autre partie d'extrémité (351a) du troisième élément (301) et qui est reliée à la première partie de connexion (51g),
**caractérisé par le fait que** :
la partie de saillie (341c) forme une première partie d'espace (W4) entre la face d'extrémité (511a) du premier élément (51) et la partie de saillie (341c) tandis que le premier élément (51) est inséré à partir de l'autre partie d'extrémité (351a) du troisième élément (301),
et la première partie de connexion (51g) est mobile par rapport au troisième élément (301) dans la direction axiale du troisième élément (301) pour ajuster la longueur de la première partie d'espace (W4) formée entre la face d'extrémité (511a) du premier élément (51) et la partie de saillie (341c), dans lequel, avec la partie maintenue (41c) du deuxième élément (41) insérée depuis la partie d'ouverture (D2, 307) du troisième élément (301), et disposée dans la première partie d'espace (W4) entre la partie de saillie (341c) et la face d'extrémité (511a) du premier élément (51), le troisième élément (301) déplace la partie de saillie (341c) par rapport au premier élément (51) de telle sorte que la partie de saillie (341c) presse la partie maintenue (41c) contre la face d'extrémité (511a) du premier élément (51), et le troisième élément (301) maintient ainsi la partie maintenue (41c) entre la face d'extrémité (511a) et la partie de saillie (341c).

2. Mécanisme de connexion (300) pour l'unité de génération/transmission de vibration ultrasonore selon la revendication 1, dans lequel le deuxième élément (41) comprend deux parties maintenues (41c) espacées l'une de l'autre dans la direction circonférentielle de la première partie d'extrémité (41a) du deuxième élément (41), et une distance (L2) entre la première et l'autre partie maintenue (41c) dans la direction diamétrale de la partie d'extrémité (41a) du deuxième élément (41) étant la longueur prescrite.

3. Mécanisme de connexion (300) pour l'unité de génération/transmission de vibration ultrasonore selon la revendication 1, dans lequel le troisième élément (301) comprend deux parties de saillie (341c) espacées l'une de l'autre dans une direction circonférentielle de la première partie d'extrémité (341a) du troisième élément (301), et le troisième élément (301) formant une seconde partie d'espace (303, 311) entre la première partie de saillie (341c) et l'autre partie de saillie (341c) dans une direction circonférentielle de la première partie d'extrémité (341a) du troisième élément (301).

4. Mécanisme de connexion (300) pour l'unité de génération/transmission de vibration ultrasonore selon la revendication 3, dans lequel la seconde partie d'espace (303, 311) est une forme telle que la partie maintenue (41c) est apte à être insérée dans ou être retirée de la seconde partie d'espace (303, 311).

5. Mécanisme de connexion (300) pour l'unité de génération/transmission de vibration ultrasonore selon la revendication 1, dans lequel le deuxième élément (41) a une partie de découpe (41m) qui est formée au côté autre partie d'extrémité (41b) du deuxième élément (41) au niveau de la partie maintenue (41c),
le troisième élément (301) a une partie d'ouverture de découpe (309) qui est formée au niveau de la partie de saillie (341c) et dans laquelle la partie de découpe (41m) est configurée pour être insérée, et
la partie de découpe (41m) est disposée dans la partie d'ouverture de découpe (309), lorsque la partie maintenue (41c) est disposée dans la partie d'ouverture (307).

6. Mécanisme de connexion (300) pour l'unité de génération/transmission de vibration ultrasonore selon la revendication 1, dans lequel la partie maintenue (41c) a une première face d'extrémité (411c) et une partie de butée (41e), et amène la partie de butée (41e) en butée avec la partie de saillie (341c).

7. Mécanisme de connexion (300) pour l'unité de génération/transmission de vibration ultrasonore selon la revendication 6, dans lequel la face d'extrémité (511a) du premier élément (51) est une première face d'extrémité,
le deuxième élément (41) comprend, au niveau de la première partie d'extrémité (41a), une seconde face d'extrémité (411a, 411c) qui vient en butée avec la face d'extrémité du premier élément (51),
la seconde face d'extrémité (411a, 411c) a au moins la première face d'extrémité (411c) de la partie maintenue (41c), et
le troisième élément (301) relie la face d'extrémité du premier élément (51) et la seconde face d'extrémité (411a, 411c) du deuxième élément (41) l'une à l'autre.

8. Mécanisme de connexion (300) pour l'unité de génération/transmission de vibration ultrasonore selon la revendication 1, dans lequel, lorsque la première partie d'extrémité (51a) du premier élément (51) est insérée dans le troisième élément (301) depuis l'autre partie d'extrémité (351a) du troisième élément (301) de telle sorte que la première partie de connexion (51g) est reliée à la seconde partie de connexion (351g), la partie de saillie (341c) est disposée à une distance supérieure à une largeur W1 de la partie maintenue faisant diamétralement saillie (41c) depuis la face d'extrémité (511a) du premier élément (51) dans la direction axiale du troisième élément (301).

9. Mécanisme de connexion (300) pour l'unité de génération/transmission de vibration ultrasonore selon la revendication 8, comprenant en outre une partie de butée (41e) qui est disposée sur la surface circonférentielle externe de la première partie d'extrémité (41a) du deuxième élément (41) et qui vient en butée avec la partie de saillie (341c) dans la direction circonférentielle du troisième élément (301) lorsque la partie maintenue (41c) est maintenue par la face d'extrémité (511a) du premier élément (51) et la partie de saillie (341c),
dans lequel, lorsque la première partie d'extrémité (51a) du premier élément (51) est insérée dans le troisième élément (301) depuis l'autre partie d'extrémité (351a) du troisième élément (301) le long de la direction axiale du troisième élément (301),
la partie maintenue (41c) est disposée entre la face d'extrémité (511a) du premier élément (51) et un plan dans lequel la partie de saillie (341c) est disposée dans la direction axiale du troisième élément (301), et
la partie de butée (41e) est disposée de manière colinéaire avec la partie de saillie dans la direction circonférentielle du troisième élément (301).

10. Mécanisme de connexion (300) pour l'unité de génération/transmission de vibration ultrasonore selon la revendication 8, comprenant en outre un mécanisme de prévention (341g, 341h, 309, 411m, 600) qui est disposé sur une surface circonférentielle interne de la première partie d'extrémité (341a) du troisième élément (301) et une surface circonférentielle externe de la première partie d'extrémité (41a) du deuxième élément (41) et qui empêche la première partie d'extrémité (41a) du deuxième élément (41), lors de l'insertion dans la première partie d'extrémité (341a) du troisième élément (301), d'être tournée par rapport au troisième élément (301) autour de l'axe du troisième élément (301),
dans lequel la partie d'ouverture (307) du troisième élément (301) est disposée dans la surface circonférentielle de la première partie d'extrémité (341a) du troisième élément (301) et est en communication avec l'intérieur du troisième élément (301) et le mécanisme de prévention (341g, 341h, 309, 411m, 600) est disposé sur la surface circonférentielle interne du troisième élément (301), et
la partie d'ouverture (307) est configurée de telle sorte que la première partie d'extrémité (41a) du deuxième élément (41) coulisse sur le troisième élément (301) par l'intermédiaire de la partie d'ouverture (307) dans la direction orthogonale à l'axe longitudinal du troisième élément (301) et est ainsi insérée dans le troisième élément (301), de telle sorte que la partie maintenue insérée (41c) est disposée entre la face d'extrémité (511a) du premier élément (51) et la partie de saillie (341c) dans la direction axiale du troisième élément (301), et de telle sorte que la rotation de la première partie d'extrémité insérée (51a) du premier élément (51) par rapport au troisième élément (301) autour de l'axe du troisième élément (301) est empêchée par le mécanisme de prévention (341g, 341h, 309, 411m, 600).

11. Procédé de production pour une unité de génération/transmission de vibration ultrasonore, étant prévu :
un mécanisme de connexion (300) selon la revendication 1,
le procédé de production comprenant un processus de connexion, le processus de connexion comprenant insérer la première partie d'extrémité (41a) du deuxième élément (41) dans le troisième élément (301) depuis la première partie d'extrémité (341a) du troisième élément (301) de telle sorte que la partie maintenue (41c) est maintenue par la face d'extrémité (511a) du premier élément (51) et la partie de saillie (341c) dans la direction axiale du troisième élément (301), insérer la première partie d'extrémité (51a) du premier élément (51) dans le troisième élément (301) depuis l'autre partie d'extrémité (351a) du troisième élément (301) de telle sorte que la seconde partie de connexion (351g) est reliée à la première partie de connexion (51g), et ainsi relier la première partie d'extrémité (51a) du premier élément (51) et la première partie d'extrémité (41a) du deuxième élément (41) l'une à l'autre.

12. Procédé de production pour l'unité de génération/transmission de vibration ultrasonore selon la revendication 11, dans lequel le mécanisme de connexion (300) comprend en outre les caractéristiques de la revendication 2.

13. Procédé de production pour l'unité de génération/transmission de vibration ultrasonore selon la revendication 11, dans lequel le mécanisme de connexion (300) comprend en outre les caractéristiques de la revendication 3.

14. Procédé de production pour l'unité de génération/transmission de vibration ultrasonore selon la revendication 11, dans lequel le mécanisme de connexion (300) comprend en outre les caractéristiques de la revendication 5.

15. Procédé de production pour l'unité de génération/transmission de vibration ultrasonore selon la revendication 11, dans lequel la face d'extrémité (511a) du premier élément (51) est une première face d'extrémité, et
le deuxième élément (41) a, au niveau de la première partie d'extrémité (41a), une seconde face d'extrémité (411a, 411c) qui vient en butée avec la face d'extrémité du premier élément (51),
le procédé de production comprenant en outre un processus de liaison du premier élément (51) et du troisième élément (301), la première partie d'extrémité (51a) du premier élément (51) étant insérée dans le troisième élément (301) depuis l'autre partie d'extrémité (351a) du troisième élément (301) tandis que la première partie de connexion (51g) est reliée à la seconde partie de connexion (351g) de telle sorte que la face d'extrémité (511a) du premier élément (51) est située à une distance supérieure à une largeur W1 de la partie maintenue (41c) depuis la partie de saillie (341c) dans la direction axiale du troisième élément (301).
